# EUROPÄISCHE PATENTSCHRIFT

(11) **EP 4 187 244 B1**
(45) Veröffentlichungstag und Bekanntmachung des Hinweises auf die Patenterteilung: **04.09.2024**
(21) Anmeldenummer: 22210610.6
(22) Anmeldetag: 30.11.2022
(51) Int. Cl.: G01N 33/24

(54) **VERFAHREN ZUR ERMITTLUNG EINES FEUCHTIGKEITSWERTES EINES BODENAREALS**
METHOD FOR DETERMINING A MOISTURE VALUE OF A GROUND AREA
PROCÉDÉ DE DÉTERMINATION D'UNE VALEUR D'HUMIDITÉ D'UNE SURFACE DE SOL

(30) Priorität: 30.11.2021 DE 102021213528
(43) Veröffentlichungstag der Anmeldung: 31.05.2023
(73) Patentinhaber: Deutsches Zentrum für Luft- und Raumfahrt e.V., 53227 Bonn (DE)
(72) Erfinder: Gewies, Stefan, 53227 Bonn (DE); Hehenkamp, Niklas, 53227 Bonn (DE); Grundhöfer, Lars, 53227 Bonn (DE); Borg, Erik, 53227 Bonn (DE)
(74) Vertreter: Brunotte, Joachim Wilhelm Eberhard

(56) Entgegenhaltungen:
- WO-A1-2008/070874
- CHADWICK DUANE G: "Integrated Measurement of Soil Moisture by Use of Radio Waves", REPORTS. PAPER 571, November 1973 (1973-11-01), XP093031627, Retrieved from the Internet <URL:https://digitalcommons.usu.edu/water_rep/571> [retrieved on 20230314]

## Beschreibung

Die Erfindung betrifft ein Verfahren und ein System zur Ermittlung von Feuchtigkeitswerten eines Bodenareals.

Die Feuchtigkeit des Bodens wird maßgeblich durch Niederschläge, Grundwasser und die Evapotranspiration, und hierbei maßgeblich durch Pflanzen, bestimmt. Ein Teil des Wassers, der durch Niederschläge in den Boden eindringt, wird entgegen der Schwerkraft vom Boden festgehalten und bleibt haften. Dieses Wasserspeichervermögen wird auch als "Feldkapazität" bezeichnet. Der Boden besitzt somit ein bestimmtes Wasserspeichervermögen.

Die Feuchtigkeit des Bodens ist ein bedeutsamer Parameter insbesondere in der Land- und Forstwirtschaft, der Geotechnik, der Bodenmechanik und der Hydrologie, hier zum Beispiel hinsichtlich Wassergehaltsänderungen oder potentiell kritischen oder gefährlichen Feuchte- oder Strömungssituationen.

In den genannten Disziplinen sind die Quantifizierung und Bewertung hydrologischer Prozesse eine wesentliche Grundvoraussetzung. Derartige Quantifizierungs- und Bewertungsprozesse können zum Beispiel betreffen:
i.) die Einschätzung der Wasserspeicherkapazität der Böden,
ii.) die Bewertung des Infiltrationsvermögens, der Tiefenversickerung und der Grundwasserneubildung zur Wasserhaushaltsbilanzierung,
iii.) die Quantifizierung und Bewertung von Bodenprozessen,
iv.) die Bewertung der Bodenfruchtbarkeit zur Einschätzung von Pflanzenwachstum, Pflanzenwasserbedarf und -verbrauch.

Bei der Analyse des hydraulischen und mechanischen Verhaltens ungesättigter Böden im Rahmen des geotechnischen Ingenieurwesens ist die Feuchtigkeit des Bodens bedeutsam.

Zum Beispiel hängen
- beim Schrumpfen bindiger Böden bei sinkenden Wassergehalten;
- beim Verlust der Standsicherheit von Hängen, begünstigt durch ergiebige Niederschläge;
- oder bei der Verflüssigung von Böden bei Bodenumlagerungen Wassergehaltsänderungen mit mechanischen Verhaltensänderungen des Bodens zusammen.

Messungen der Feuchtigkeit des Bodens zielen auf die Ermittlung des Bodenwassergehaltes, der zu einem spezifischen Zeitpunkt im Boden enthalten ist, ab. Sie erfolgen in der Regel mittels Bodenfeuchtesensoren. Im Folgenden werden die verschiedenen, bekannten Messmethoden zur Ermittlung von Feuchtigkeitswerten des Bodens dargestellt.

Handprobe: Eines der ältesten Messverfahren, um die Feuchtigkeit des Bodens zu bestimmen, ist eine Handprobe des Bodens. Wie feucht der Boden ist, wird durch die Bodenfeuchte ausgedrückt. Qualitativ kann die Bodenfeuchte in verschiedene Stufen von "nass" bis "trocken" eingeteilt werden. In vielen Fällen ist jedoch eine quantitative Bestimmung der Bodenfeuchte erforderlich.

Gravimetrische Bestimmung des Wassergehaltes: Die gravimetrische Bestimmung des Wassergehaltes ist ein direktes Messverfahren. Dazu wird eine Bodenprobe bekannten Volumens gewogen, anschließend bei 105°C getrocknet und erneut gewogen. Aus der Differenz zwischen Feuchtemasse und Trockenmasse der Probe kann dann die Masse des in der Probe gebundenen Wassers ermittelt werden. Um anschließend den volumetrischen Wassergehalt zu bestimmen, wird die Masse des Wassers über die Dichte in das Volumen des Wassers umgerechnet.

Kapazitive Feuchtigkeitsmessung: Ein kapazitiver Feuchtigkeitsmesser erfasst die elektrische Kapazität der direkten Bodenumgebung und lässt so Rückschlüsse auf die Feuchtigkeit des Bodens zu. Die Dielektrizitätszahl sowie weitere Parameter (Bodenart, Bodendichte, Salzgehalt etc.) beeinflussen das Messergebnis, so dass eine regelmäßige Kalibrierung sinnvoll ist.

Impedanzspektroskopie: Die Impedanzspektroskopie ermittelt die Feuchtigkeit des Bodens anhand einer Bestimmung des Wechselstromwiderstandes in Abhängigkeit von der Frequenz.

Tensiometer sind z. B. Keramikkerzen, die kontinuierlich die Bodenfeuchtebestimmung durch Messung der Saugspannung des Bodens ermöglichen. Dabei gilt: Ein trockener Boden weist größere Potentiale der Adsorptions- und Kapillarkräfte als ein feuchter Boden auf. Die Messung ist oft aussagekräftiger als indirekte Messmethoden aber die Sensoren können den hydraulischen Kontakt zur Bodenmatrix verlieren (z. B. durch Frost) und benötigen eine regelmäßige Wartung.

Feuchtigkeitsbestimmung mittels "Time Domain Reflectometry" (TDR): Die Messung der Feuchtigkeit mittels Time Domain Reflectometry ermittelt einen durchschnittlichen Feuchtigkeitswert im Boden entlang eines Sensors. Der Feuchtigkeitswert wird durch die Laufzeitmessung eines am feuchten Boden reflektierten elektrischen Signals bestimmt. Der punktuelle Feuchtegehalt entlang eines Sensors lässt sich durch Anpassungen des Sensors, komplexe Algorithmen und ein entsprechendes Rekonstruktionsverfahren bestimmen.

Feuchtigkeitsbestimmung mittels "Frequency-Domain-Reflectometry" (FDR): Die Frequency-Domain-Reflectometry misst den Bodenwassergehalt mittels eines kontinuierlich erzeugten (z. B. mittels Oszillators) elektromagnetischen Signals konstanter Frequenz und Amplitude, das über eine Hochfrequenzleitung (z. B. mittels Messstäbe) ausgesandt wird. So entsteht um die Stäbe, die im Boden eingebaut sind, ein schwingendes elektromagnetisches Feld. Der Boden dient als Dielektrikum und setzt der Ausbreitung des elektromagnetischen Feldes einen Widerstand entgegen. Die Rekonstruktion der Feuchtigkeit findet über die Auswertung der Laufzeit des reflektierten Signals statt.

Feuchtigkeitsbestimmung mittels Gammastrahlung: Die fernerkundungsgestützte Messung der Bodenfeuchtigkeit mittels Gammastrahlung nutzt die Strahlungsdifferenz von natürlichen terrestrischen Gammastrahlungen zwischen feuchten und trockenen Bodenbereichen. Dabei wird davon ausgegangen, dass das Wasser in den oberen Bodenschichten die Gammastrahlung darunterliegender Bodenschichten abschwächt, so dass die Strahlung bei feuchten Bodenbereichen geringer als bei trockenen Bodenbereichen ist.

Cosmic-ray Neutron Survey (CRN) ist eine weitere zerstörungsfreie Technik zur großräumigen Bestimmung der Bodenfeuchtigkeit. Das Verfahren beruht auf dem Vorhandensein kosmischer Strahlung auf der Erde und der Erzeugung von Neutronen. Diese Neutronen interagieren mit der Atmosphäre und dem Boden, wobei Kollisionen mit den im Bodenwasser enthaltenen Wasserstoffkernen zum Energieverlust der Neutronen führt, so dass diese mittels Neutronensonden gemessen und auf den Bodenwassergehalt geschlossen werden kann.

Bodenfeuchtigkeitsbestimmung im sichtbaren (VIS), nahen (NIR) und mittleren Infrarotbereich (MIR): Die Fernerkundung im VIS, NIR und MIR nutzt den geringeren Albedowert feuchten Bodens im Vergleich zu trockenem Boden und nutzt die Differenz beider Werte zur Feuchtigkeitsbestimmung. Eine Reihe von Einflussfaktoren, wie organisches Material, Oberflächenrauheit, Bodentextur, Einfallswinkel der Strahlung, Bodenfarbe, Vegetationsbedeckung sowie die starke Variabilität der Bodenfeuchtigkeit stellen hierbei Schwierigkeiten dar.

Thermales Infrarot (TIR): Das thermale Infrarot (TIR) ermöglicht einen theoretisch fundierten Ansatz zur Bodenfeuchtemessung. Unter Berücksichtigung aller meteorologischen Eingangsdaten hängt die Oberflächentemperatur des Bodens u. a. von der thermischen Trägheit ab. Diese ist wiederum abhängig von der Wärmeleitfähigkeit und der spezifischen Wärmespeicherkapazität des Bodens. Die thermische Trägheit ist stark durch die Bodenfeuchtigkeit und nur schwach durch das Sand-Ton-Verhältnis beeinflusst.

Mikrowellen (MW): Im Bereich der Mikrowellen spielt die Bodenfeuchtigkeit eine wichtige Rolle. Das gilt sowohl für die passive MW-Radiometrie als auch für die Radarfernerkundung. Im Fall der Radiometrie wird die Dominanz des Bodenwassers auf die Dielektrizitätskonstante des Bodens und damit auf dessen Emissionsvermögen ausgenutzt. Mit zunehmender Bodenfeuchtigkeit kann mit einer Abnahme der Strahlungstemperatur des Bodens gerechnet werden. Im Fall der RADAR-Fernerkundung wirken prinzipiell die gleichen Bodenparameter wie im Fall der passiven Radiometrie, dies sind die Dielektrizitätskonstante und die Oberflächenrauheit des Bodens. Es kann aber generell davon ausgegangen werden, dass mit zunehmender Bodenfeuchtigkeit ein Anstieg des Reflexionsvermögens verbunden ist.

Anders als bei anderen Umweltparametern wie Niederschlag, relative Luftfeuchtigkeit und Temperatur werden aufgrund der aufwendigen und schwierigen Messverfahren der Bodenfeuchtigkeit in Deutschland keine flächendeckenden Informationen mithilfe von Messstationen erhoben. Daher wird z. B. durch den Deutschen Wetterdienst (DWD, Bundesoberbehörde) die aktuelle Bodenfeuchtigkeitssituation in Deutschland in der Schicht von 0 bis 60 cm unter Gras bei lehmigem Sand (leichter Boden) oder sandigem Lehm (schwerer Boden) als simulierte Bodenfeuchtigkeit flächendeckend angegeben. Ein Problem punktueller Bodenfeuchtigkeitsmessung bzw. flächendeckender Bodenfeuchtigkeitssimulationen ist die Tatsache, dass der Bodenwassergehalt von der jeweilig vorherrschenden Bodenart und dem Zustand der jeweiligen Bodenart (z. B. der Lagerungsdichte) bestimmt wird. Allerdings resultiert insbesondere daraus eines der wesentlichen Probleme bei der Simulation, da insbesondere die küstennahen Regionen Deutschlands durch ein kleinräumlich stark differenziertes Verbreitungsmuster der Bodenarten charakterisiert ist.

Die vorgestellten Methoden zur Messung der Bodenfeuchtigkeit zielen grundsätzlich auf die Ermittlung des Bodenwassergehaltes, der zu einem spezifischen Zeitpunkt im Boden enthalten ist. Viele der vorgestellten Methoden basieren außerdem auf punktuellen Messungen. Keines der vorgestellten Verfahren kann flächenhaft große Gebiete kontinuierlich abdecken. Es handelt sich entweder um Punktmessungen, die zwar zeitlich kontinuierlich erfolgen können, deren räumliche Repräsentativität jedoch begrenzt ist oder es handelt sich um flächenhafte Messungen, die jedoch nicht zeitlich kontinuierlich zur Verfügung stehen oder nur mit einem sehr hohen technischen und finanziellen Aufwand zeitlich kontinuierlich realisierbar wären. Typischerweise kommen die vorgestellten Methoden für das Überwachen der Bodenfeuchte in der Landwirtschaft (z. B. zur Unterstützung einer Bewässerungssteuerung) oder für Forschungszwecke oder für geohydrologische Zwecke zum Einsatz.

Chadwick Duane G: "Integrated Measurement of Soil Moisture by Use of Radio Waves", Reports. Paper 571, November 1973 (1973-11), XP093031627, URL: https://digitalcommons.usu.edu/water_rep/571 beschreibt, dass die Bodenfeuchte durch Messung der Dämpfung von vertikal polarisierten Oberflächen-Radiowellen ermittelt werden kann, die sich von einem Sender zu einem Empfänger über den Boden ausbreiten.

WO 2008/070874 A1 beschreibt, wie die Bodenfeuchtigkeit durch Messung der Veränderung der elektrischen Leitfähigkeit in der Nähe der Erdoberfläche unter Verwendung von sich an der Oberfläche ausbreitenden elektromagnetischen Feldern geschätzt werden kann. Es wird ein Verfahren zur Schätzung der Bodenfeuchtigkeit in Oberflächennähe angegeben, einschließlich der Messung von Signalen einer elektromagnetischen Bodenwelle, die sich zwischen einem oder mehreren Empfangselementen ausbreitet. Das Verfahren schließt ferner ein: das Bestimmen einer Übertragungscharakteristik proportional zu einer durchschnittlichen elektrischen Leitfähigkeit zwischen Paaren von Orten ein und das Bestimmen der geschätzten Bodenfeuchtigkeit in einem oder mehreren Bereichen, die aus der Analyse der bestimmten elektrischen Leitfähigkeit zwischen Paaren von Orten und einer vorbestimmten regionalen Beziehung zwischen elektrischer Leitfähigkeit und Bodenfeuchtigkeit abgeleitet ist.

Es ist eine Aufgabe der vorliegenden Erfindung, Bodenfeuchtigkeitswerte insbesondere von großen Bodenarealen zeitlich kontinuierlich, zuverlässig, flächendeckend und auf einfache, preisgünstige und/oder automatisierte Art und Weise zu bestimmen. Eine weitere Aufgabe der vorliegenden Erfindung ist es, eine Verbesserung und eine Detaillierung der räumlichen und zeitlichen Charakterisierung der Bodenfeuchtigkeit von großen Bodengebieten zu erreichen, insbesondere während einer Vegetationsperiode.

Die beigefügten Patentansprüche definieren den Schutzumfang.

Wenn von "Bodenarealen" oder dem "Boden" gesprochen wird, können insbesondere Erdbodenareale oder der Erdboden gemeint sein. Es können auch insbesondere Areale der Erdoberfläche oder die Erdoberfläche gemeint sein. Bodenwellen werden allgemeinhin als "Bodenwellen" bezeichnet, auch wenn sie sich an einer Wasseroberfläche oder an einer Eisoberfläche verbreiten. Allgemein verbreiten sich Bodenwelle an der unteren Grenzschicht zur Luft. Demgemäß kann unter "Boden" auch eine Wasseroberfläche oder eine Eisoberfläche verstanden werden. Ein "Bodenareal" kann ein Areal einer Wasseroberfläche oder einer Eisoberfläche sein oder ein solches Areal (zum Beispiel neben einem festen Erdbodenareal) aufweisen. Ein Bereich unter Wasser, zum Beispiel am Grund eines Sees oder Meeres, wird als "Seegrund" oder "Meeresgrund" oder allgemein "Grund" bezeichnet.

Wenn von "Bodenarealen" oder dem "Boden" gesprochen wird, kann und sollte bei der Definition berücksichtigt werden, dass die Ausbreitung elektromagnetischer Wellen im Bereich der Erdatmosphäre grundsätzlich über eine Bodenwelle, eine Raumwelle und eine direkte Welle erfolgen kann. Da sich abgestrahlte Bodenwellen parallel zur Erdoberfläche und mit zunehmendem Halbkugelradius in alle Richtungen der Erdoberfläche ausbreiten, durchquert die Bodenwelle auf ihrem Ausbreitungsweg unterschiedliche Medien (z. B. festen Erdboden, Permafrost, Eis, Wasser).

Unter einem "Bodenareal" oder dem "Boden" kann demzufolge ein Ausschnitt der Erdoberfläche verstanden werden, der eine direkte Grenzschicht zur Atmosphäre darstellt oder umfasst, wobei seine Mächtigkeit (Dicke) wesentlich durch eine Frequenz einer diesen Ausschnitt durchdringenden Bodenwelle definiert wird. Mit steigender Frequenz nimmt eine Eindringtiefe eines durch die Bodenwelle verursachten elektrischen Feldes in den Boden bzw. das Bodenareal ab. Dadurch wird der für die Stromleitung zur Verfügung stehende effektive Querschnitt geringer, so dass eine Reichweite der Bodenwelle abnimmt.

Eine Unterscheidung der Medien auf dem Ausbreitungsweg der Bodenwelle kann mit Lageinformationen (bevorzugt Karten) aufgeklärt werden.

Der Begriff der Bodenwelle kann allgemein insbesondere umfassen, dass eine elektromagnetische Welle gemeint ist, die sich an der Grenzschicht der Erdoberfläche (Erdboden, Eis oder Wasser) und Luft ausbreitet.

Gemäß einem Grundgedanken der vorliegenden Erfindung kann mithilfe einer Sendeeinrichtung ein elektromagnetisches Signal als Bodenwelle von einer ersten Position ausgesendet werden. Die erste Position kann zum Beispiel ein Ort am Boden oder auf dem Boden oder auf der Bodenoberfläche oder über dem Boden oder unter dem Boden sein. Die erste Position kann sich über einem Meeresgrund oder einem Seegrund befinden, sodass sich zwischen dem Meeresgrund oder dem Seegrund und der ersten Position Wasser befinden kann, zum Beispiel, wenn die erste Position sich auf oder an der Wasseroberfläche eines Sees oder eines Meeres befindet.

Die Sendeeinrichtung - insbesondere zum Beispiel ein Sendeinstrument der Sendeeinrichtung, zum Beispiel eine Antenne - kann insbesondere in, an oder bei der ersten Position, zum Beispiel am oder auf dem Boden, vorgesehen sein, zum Beispiel in einem vertikalen Abstand von nicht mehr als 10 Metern, bevorzugt nicht mehr als 1 Meter, und/oder mit dem Boden elektrisch leitend verbunden sein. Insbesondere kann sich die Sendeeinrichtung mindestens zum Teil in den Boden erstrecken. Die Sendeeinrichtung kann insbesondere eine Antenne aufweisen. Die Sendeeinrichtung, insbesondere zum Beispiel ein Teil der Sendeeinrichtung, zum Beispiel eine (Sende-)Antenne der Sendeeinrichtung, kann direkt auf oder in der ersten Position angeordnet sein. Die Sendeeinrichtung kann auch oberhalb (zum Beispiel mindestens 1 Zentimeter oder mindestens 1 Dezimeter oder mindestens 1 Meter oberhalb) der ersten Position angeordnet sein oder unterhalb (zum Beispiel sich mindestens teilweise in den Boden erstreckend) der ersten Position angeordnet sein. Die Sendeeinrichtung, insbesondere zum Beispiel eine Antenne der Sendeeinrichtung, kann sich zum Beispiel mindestens zum Teil in einem Umkreis von 2 oder 5 oder 10 Metern von der ersten Position befinden. Eine Position der Sendeeinrichtung, zum Beispiel ein Standort, zum Beispiel ein Standort der Antenne der Sendeeinrichtung, kann die erste Position definieren. Die erste Position kann zum Beispiel durch Koordinaten, insbesondere geographische Koordinaten, angegeben sein.

Das Senden des elektromagnetischen Signals derart, dass es sich als Bodenwelle verbreitet, kann zum Beispiel dadurch erreicht werden, dass die Sendeeinrichtung zum Beispiel in einem vertikalen Abstand von mehreren Dekametern, oder (vorzugsweise) in einem vertikalen Abstand von 1 bis 10 Metern von der Erdoberfläche angeordnet ist und/oder mit dem Boden elektrisch leitend verbunden ist und/oder sich die Sendeeinrichtung mindestens zum Teil in den Boden erstreckt, vorzugsweise einige Zentimeter (z. B. 10 bis 50) bis zu mehreren Metern (z. B. 1 bis 10) in den Boden erstreckt. Eine geeignete Länge kann abhängig von der Frequenz der Bodenwelle sein. Eine Länge einer Antenne, die zur Sendeeinrichtung gehört, oder eines Antennenteils oberhalb des Bodens oder unterhalb des Bodens, kann in Abhängigkeit von der Frequenz bestimmt sein.

Eine Bodenwelle kann insbesondere im Frequenzbereich von 30 kHz bis 30 MHz liegen. Das elektromagnetische Signal kann daher (z. B. am Sender oder am Empfänger gemessen) eine Frequenz in diesem Intervall aufweisen. Es ist nicht ausgeschlossen, dass das elektromagnetische Signal eine Frequenz unterhalb dieses Intervalls oder oberhalb dieses Intervalls aufweist, solange ihre Eigenschaft als Bodenwelle über eine gewünschte Distanz bestehen bleibt. Das elektromagnetische Signal muss insbesondere in der gewünschten Distanz - die der Abstand zwischen der ersten Position und einer zweiten Position sein kann - empfangbar sein und die gewünschte Distanz als Bodenwelle zurückgelegt haben.

Anders formuliert kann das elektromagnetische Signal eine Frequenz aufweisen, die
- in dem Frequenzbereich der Mittelwellen oder
- in dem Frequenzbereich der Langwellen oder
- nahe unterhalb des Frequenzbereichs der Langwellen oder
- in dem Frequenzbereich der Kurzwellen oder
- nahe oberhalb des Frequenzbereichs der Kurzwellen liegt.

Natürliche Materialien (z.B. Boden, Permafrost, Eis, Wasser) weisen nur eine begrenzte Leitfähigkeit auf, so dass sich darin ausbreitenden elektromagnetischen Feldern Energie (Erdverluste) entzogen wird. D.h., je geringer Leitfähigkeiten eines Ausschnitts der Erdoberfläche auf einem Ausbreitungsweg eines elektromagnetischen Felds sind, desto kleiner ist eine Reichweite einer Bodenwelle.

Ein zu untersuchender Teilbereich eines Bodenareals kann kürzer sein als der Abstand zwischen der ersten Position und der zweiten Position, zum Beispiel, wenn der Sender oder der Empfänger auf einer Vorrichtung auf dem Wasser befindlich ist und ein Landabschnitt, der einen zu untersuchenden Teilbereich eines Bodenareals oder einen zu untersuchenden Teilbereich eines Bodenpfads enthält, erst in Entfernung von dem Sender oder dem Empfänger beginnt.

An der zweiten Position kann das elektromagnetische Signal empfangen werden. In dieser Beschreibung wird davon gesprochen, dass "das" elektromagnetische Signal an der zweiten Position empfangen wird. Selbstverständlich hat sich das elektromagnetische Signal auf seinem Weg von der ersten Position zu der zweiten Position verändert. Es ist jedoch möglich, es als dasjenige Signal zu identifizieren (oder dies ist aus zumindest einem Grund bekannt und insbesondere eindeutig), welches an der ersten Position gesendet wurde. Die zweite Position kann zum Beispiel ein Ort am Boden oder auf dem Boden oder auf der Bodenoberfläche oder über dem Boden oder unter dem Boden sein. Die zweite Position kann sich über einem Meeresgrund oder einem Seegrund befinden, sodass sich zwischen dem Meeresgrund oder dem Seegrund und der zweiten Position Wasser befinden kann, zum Beispiel, wenn die zweite Position sich auf oder an der Wasseroberfläche eines Sees oder eines Meeres befindet.

Zwischen der ersten Position und der zweiten Position kann sich ein zu untersuchendes Bodenareal oder ein zu untersuchender Bodenpfad erstrecken. Das Bodenareal oder der Bodenpfad kann sich zwischen der ersten Position und der zweiten Position auf dem und/oder entlang dem Großkreis befinden, der durch die erste Position und die zweite Position definiert wird. Eine Distanz zwischen der ersten Position und der zweiten Position kann zum Beispiel
- mindestens 10 Meter betragen oder in der Größenordnung von mehreren (z. B. 1-10) Dekametern liegen oder
- mindestens 100 Meter betragen oder in der Größenordnung von mehreren (z. B. 1-10) Hektometern liegen oder
- mindestens 1 km betragen oder in der Größenordnung von mehreren (z. B. 1-10) Kilometern liegen oder
- mindestens 10 km betragen oder in der Größenordnung von mehreren (z. B. 1-10) 10 km liegen oder
- mindestens 100 km betragen oder in der Größenordnung von mehreren (z. B. 1-10) 100 km liegen oder
mindestens 1000 km betragen oder in der Größenordnung von mehreren (z. B. 1-10) 1000 km liegen.

Ein zu untersuchender Teilbereich des Bodenareals muss in seiner Längsausdehnung nicht die gesamte Distanz zwischen der ersten Position und der zweiten Position aufweisen, insbesondere auch dann, wenn über die gesamte Distanz Boden (und daher kein Wasser, insbesondere ein See oder ein Meer) die Oberfläche bildet. Anders ausgedrückt kann der Teilbereich in seiner Längsausdehnung kürzer sein als die Distanz zwischen der ersten Position und der zweiten Position.

Die Empfangseinrichtung - insbesondere zum Beispiel ein Empfangsinstrument der Empfangseinrichtung, zum Beispiel eine Antenne - kann insbesondere in, an oder bei der zweiten Position am Boden vorgesehen sein, zum Beispiel in einem vertikalen Abstand von mehreren Dekametern (z. B. 1 bis 10), vorzugsweise aber 1 bis 10 Meter von der Erdoberfläche, und/oder mit dem Boden elektrisch leitend verbunden sein. Insbesondere kann sich die Empfangseinrichtung mindestens zum Teil in den Boden erstrecken. Die Empfangseinrichtung kann insbesondere eine Antenne sein. Die Empfangseinrichtung, insbesondere zum Beispiel ein Teil der Empfangseinrichtung, zum Beispiel eine (Empfangs-)Antenne der Empfangseinrichtung, kann direkt auf oder in der zweiten Position angeordnet sein. Die Empfangseinrichtung kann auch oberhalb (zum Beispiel im Zentimeterbereich [z. B. 1 bis 100] bis mehrere Meter oberhalb der Erdoberfläche, z. B. 1 bis 100) der zweiten Position angeordnet sein oder unterhalb (zum Beispiel sich mindestens teilweise in den Boden erstreckend) der zweiten Position angeordnet sein. Die Empfangseinrichtung, insbesondere zum Beispiel eine Antenne der Empfangseinrichtung, kann sich zum Beispiel mindestens zum Teil in einem Umkreis von einigen Dezimetern (z. B. 2, 5 oder 10), oder einigen Metern (z. B. 2, 5, oder 10) bis einigen Dekametern (z. B. 2, 5 oder 10) von der zweiten Position befinden. Eine Position der Empfangseinrichtung, zum Beispiel ein Standort, zum Beispiel ein Standort der Antenne der Empfangseinrichtung, kann die zweite Position definieren. Die zweite Position kann zum Beispiel durch Koordinaten, insbesondere geographische Koordinaten, angegeben sein.

Die zweite Position kann alternativ auch ein Ort auf oder an einer Wasseroberfläche sein. Dort kann sich die Empfangseinrichtung befinden. Zwischen der ersten Position und der zweiten Position kann sich ein zu untersuchender Teilbereich des Bodenareals oder ein zu untersuchender Teilbereich des Bodenpfads lediglich in einem Abschnitt befinden. Es ist auch nicht ausgeschlossen, dass sich die erste Position (und die Sendeeinrichtung) alternativ oder zusätzlich auf oder an einer Wasseroberfläche befindet und sich ein zu untersuchender Teilbereich des Bodenareals oder ein zu untersuchender Teilbereich des Bodenpfads zwischen der ersten und der zweiten Position in einem Landabschnitt befindet. Die erste Position und die zweite Position können sich an Orten befinden, die zu manchen Zeiten eine Wasseroberfläche und zu anderen Zeiten eine Landoberfläche aufweisen. Solche Orte können zum Beispiel Orte sein, die dem Einfluss von Ebbe und Flut ausgesetzt sind. Der Gegenstand der beigefügten Patentansprüche betrifft in aber in jedem Fall eine Situation, bei der das Bodenareal eine Mehrzahl von Teilbereichen aufweist, die hintereinander liegend angeordnet sind. Da diese Teilbereiche jeweils eine gemeinsame Grenze mit einem anderen Boden-Teilbereich oder zumindest eine Grenze zu einem anderen Bereich wie zum Beispiel an einem Seeufer oder einer Meeresküste haben, kann die Vorgehensweise auf Basis dieser hintereinander liegenden Boden-Teilbereiche und anderen Teilbereiche als Finite-Elemente-Verfahren bezeichnet werden. Jedes der finiten Elemente entspricht einem Teil-Abschnitt entlang einer direkten Verbindungslinie von der ersten Position zu der zweiten Position. In der Praxis kann es vorkommen, dass es nicht nur zwei Boden-Teilbereiche sondern eine sehr viel höhere Anzahl wie zum Beispiel mehr als zehn oder mehr als fünfzig Boden-Teilbereiche zwischen der ersten Position und der zweiten Position gibt.

Es werden Werte von physikalischen Eigenschaften des empfangenen elektromagnetischen Signals ermittelt, zum Beispiel durch Messung und/oder Berechnung. Ein Wert einer der physikalischen Eigenschaft ist gemäß den Ansprüchen ein Phasenwert des empfangenen elektromagnetischen Signals. Ein Wert einer möglichen weiteren physikalischen Eigenschaft ist die Laufzeit des elektromagnetischen Signals von der ersten Position zur zweiten Position. "Phase" kann allgemein als Synonym zu "Phasenwinkel" verstanden werden. Die Angabe des Phasenwerts kann in allen möglichen Einheiten erfolgen, die einen Phasenwert kennzeichnen können (z. B. Grad, rad oder Sekunden) oder aus denen der Phasenwert abgeleitet werden kann. Der Phasenwert kann sich allgemein zum Beispiel auf eine elektrische oder eine magnetische Feldkomponente des empfangenen elektromagnetischen Signals beziehen. Der Phasenwert kann auch bestimmt werden, indem von dem empfangenen elektromagnetischen Signal in der Empfangseinheit hervorgerufene Ströme oder Spannungen gemessen werden. Ferner wird gemäß den Ansprüchen zusätzlich als weitere physikalische Eigenschaften die Amplitude eines Feldes (insbesondere des elektrischen Wechselfeldes und/oder des magnetischen Wechselfeldes) des elektromagnetischen Signals bestimmt. Die Amplitude ist insbesondere ein Maß für die Dämpfung des elektromagnetischen Signals, die es auf seinem Weg von der ersten zu der zweiten Position erfährt.

Eine Veränderung von Phasenwerten oder von Werten der Laufzeit wird insbesondere verursacht durch eine Veränderung elektrischer Eigenschaften (insbesondere: elektrische Leitfähigkeit, Impedanz oder dielektrische Eigenschaften, insbesondere relative Dielektrizitätskonstante bzw. Permittivität) des Bodenareals oder des Bodenpfades, das/der zwischen der ersten Position und der zweiten Position liegt. Daher kann eine ermittelte Veränderung von Phasenwerten oder von Werten der Laufzeit auf eine Veränderung elektrischer Eigenschaften schließen lassen. Die Veränderung eines Phasenwerts oder eines Wertes der Laufzeit kann sich insbesondere auf einen Referenzwert derselben physikalischen Größe, also ebenfalls der Phase oder der Laufzeit, beziehen.

Das elektromagnetische Signal kann auch nicht-kontinuierlich sein, also zum Beispiel Lücken oder Sprünge hinsichtlich seiner Amplitude aufweisen. Beispielsweise kann das elektromagnetische Signal gepulst sein oder gepulste Anteile aufweisen. Ein Wert einer der physikalischen Eigenschaften kann zum Beispiel eine (gemessene) Zeitdifferenz (oder eine Laufzeit) eines Anteils des elektromagnetischen Signals - zum Beispiel einer Flanke, eines Nulldurchgangs oder eines Peaks oder eines anderen charakteristischen Merkmals des elektromagnetischen Signals - zwischen dem Senden mithilfe der Sendeeinrichtung und dem Empfangen mithilfe der Empfangseinrichtung sein.

Das elektromagnetische Signal kann alternativ oder zusätzlich verschiedene Frequenzen aufweisen. Beispielsweise kann das elektromagnetische Signal verschiedene Frequenzen aufweisen, die zeitlich nacheinander auftreten. Zum Beispiel können verschiedene Frequenzen in kontinuierlicher Form derart auftreten, dass die Frequenzen kontinuierlich oder schrittweise ansteigen (z. B. in Form einer Rampe) oder abfallen. In einer solchen Ausgestaltung des erfindungsgemäßen Verfahrens können mehrere Werte der physikalischen Eigenschaften (z. B. Phasenwerte oder Werte von Laufzeiten) bei unterschiedlichen Frequenzen nacheinander ermittelt werden. Eine solche Ausgestaltung ist vorteilhaft, weil das Bodenareal so in unterschiedlichen Tiefen (Eindringtiefen des Signals) untersucht werden kann.

Das elektromagnetische Signal kann alternativ oder zusätzlich (also in Kombination mit verschiedenen Frequenzen, die nacheinander auftreten) gleichzeitig verschiedene Frequenzanteile aufweisen. In einer solchen Ausgestaltung des erfindungsgemäßen Verfahrens können mehrere Werte der physikalischen Eigenschaften (z. B. Phasenwerte oder Werte von Laufzeiten) bei unterschiedlichen Frequenzen gleichzeitig ermittelt werden. Eine solche Ausgestaltung ist vorteilhaft, weil das Bodenareal so in unterschiedlichen Tiefen (Eindringtiefen des Signals) untersucht werden kann. Eine Untersuchung kann besonders schnell vorgenommen werden, weil die Werte der physikalischen Eigenschaften gleichzeitig oder nahezu gleichzeitig ermittelt werden können.

Die Veränderung der elektrischen Eigenschaften oder mindestens einer der elektrischen Eigenschaften des Bodenareals oder des Bodenpfades lässt wiederum auf eine Veränderung der Bodenfeuchtigkeit schließen. Daher kann von einer Veränderung des Phasenwertes oder des Wertes der Laufzeit oder, allgemein gesprochen, von einer Veränderung des Wertes der physikalischen Eigenschaft des elektromagnetischen Signals auf eine Veränderung der Bodenfeuchtigkeit des Bodenareals bzw. entlang des Bodenpfades geschlossen werden.

Die Breite, die auch als Querausdehnung des Bodenareals bezeichnet werden kann, braucht nicht genau bestimmbar zu sein oder bestimmt zu werden. In der Recommendation P527-3 (03/1992) "Electrical characteristics of the surface of the Earth" der International Telecommunication Union (ITU) wird unter Punkt 3.3 beschrieben, dass die Energie von elektromagnetischen Signalen sich allgemein nicht nur auf direktem Pfad zwischen einem Sender und einem Empfänger ausbreitet, sondern dass als Breite zu beiden Seiten jeweils eine Fresnel-Halbwellen-Zone angenommen werden kann. Hierin liegt eine Möglichkeit, die Breite des Bodenareals, die sich dann von dem direkten Pfad seitlich zu beiden Seiten jeweils um eine Fresnel-Halbwellen-Zone lang erstreckt (Gesamtbreite zwei Fresnel-Halbwellen-Zonen), in sinnvoller Art und Weise zu bestimmen oder festzulegen.

Die Breite wird allgemein durch die physikalischen Gegebenheiten der Feuchtigkeitsermittlung mithilfe des vorgestellten Verfahrens implizit begrenzt. Sie kann - und wird bei praktischer Umsetzung des erfindungsgemäßen Verfahrens - in den meisten Fällen deutlich schmaler sein als die Längsausdehnung des Bodenareals in der Richtung zwischen der ersten Position und der zweiten Position. Daher wird der Begriff des Bodenpfads und eine Ermittlung eines Feuchtigkeitswerts entlang des Bodenpfads als eine Alternative zum beanspruchten Begriff des Bodenareals vorgestellt, wobei eine geringe Breite des Bodenpfads angenommen wird. Ein Bodenareal, das in Querausdehnung deutlich schmaler ist als in Längsausdehnung, wobei die Querausdehnung nicht genau bestimmbar ist oder nicht genau bestimmt wird, kann trotzdem in sinnvoller Art und Weise zur Ermittlung eines Feuchtigkeitswerts genutzt werden. Ein ermittelter Feuchtigkeitswert kann zum Beispiel als repräsentativer und/oder durchschnittlicher Feuchtigkeitswert eines zu definierenden Bodengebiets dienen, das breiter ist als das durch das erfindungsgemäße Verfahren real tomographisch erfasste Bodenareal selbst. Dieses Bodengebiet kann letztendlich am Ende selbst als das Bodenareal definiert werden, zu dem der Feuchtigkeitswert zugehörig ist.

Ein zu untersuchendes Bodenareal oder ein zu untersuchender Bodenpfad können - je nach Wellenlänge - Tiefenwerte in der Größenordnung von mehreren Zentimetern, mehreren Dezimetern oder mehreren Metern aufweisen, die den Tiefen entsprechen, in denen sich das elektromagnetische Signal als Bodenwelle messbar fortpflanzt. Eine genaue Bestimmung der Tiefe ist nicht erforderlich.

Gemäß Anspruch 1 wird ein Verfahren mit folgenden und weiteren Merkmalen vorgeschlagen: Es dient der Ermittlung eines Feuchtigkeitswertes eines Bodenareals, das sich zwischen einer ersten Position und einer zweiten Position befindet, und weist auf:
- Senden eines elektromagnetischen Signals an der ersten Position mithilfe einer Sendeeinrichtung derart, dass es sich als Bodenwelle verbreitet;
- Empfangen des elektromagnetischen Signals an der zweiten Position mithilfe einer Empfangseinrichtung;
- Ermitteln je eines Wertes von zumindest zwei physikalischen Eigenschaften des elektromagnetischen Signals;
- Ermitteln des Feuchtigkeitswertes des Bodenareals mithilfe der Werte der physikalischen Eigenschaften;
   wobei
- das Bodenareal eine Mehrzahl von Teilbereichen aufweist, die bezüglich einer entlang der Bodenoberfläche des Bodenareals verlaufenden direkten Verbindungslinie der ersten Position mit der zweiten Position hintereinander liegend angeordnet sind,
- basierend auf einem Modell des Bodenareals, welches jeweils zumindest eine elektrische physikalische Größe der Teilbereiche in Abhängigkeit von den zumindest zwei physikalischen Eigenschaften des elektromagnetischen Signals an der zweiten Position beschreibt, der Feuchtigkeitswert jeweils der Mehrzahl der Teilbereiche des Bodenareals ermittelt wird.

Diese Vorgehensweise ermöglicht es somit, Teilbereiche von Bodenarealen zwischen der ersten und der zweiten Position hinsichtlich ihrer Feuchtigkeit zu erfassen, die unterschiedliche Bodeneigenschaften haben. Insbesondere können so Bodenareale mit verschiedenen Bodenarten untersucht werden. Insbesondere können die Teilbereiche jeweils aus einer einzigen Bodenart bestehen. In der Praxis allerdings kann es sich dabei um eine Näherung einer idealisierten Annahme handeln, d. h. auch innerhalb des Teilbereichs können die Eigenschaften variieren, wenn auch in geringerem Umfang. Für die Unterteilung des Bodenareals in verschiedene Teilbereiche können insbesondere Informationen aus für viele geographische Gebiete verfügbare Bodenkarten entnommen werden. Insbesondere kann ein Bodenmodell verwendet werden, um die physikalischen Eigenschaften des Bodens zu berechnen und/oder zu berücksichtigen. Das Bodenmodell stellt den Zusammenhang zwischen den aus den Bodenkarten entnommenen Werten der Bodenarten entlang des Ausbreitungspfades unter Einbeziehung des (insbesondere volumetrischen) Wasseranteils und der Temperatur wie ausführlich beschreiben in ITU 527-6, insbesondere Abschnitt 5.2, und den physikalischen Eigenschaften (elektrische Leitfähigkeit und Dielektrizität) des Bodens her.

Das Modell kann z. B. außer dem Bodenmodell auch ein so genanntes Ausbreitungsmodell enthalten, welches für die verschiedenen Teilbereiche den Zusammenhang zwischen der zumindest einen elektrischen physikalischen Größe der Teilbereiche (zum Beispiel elektrische Bodenleitfähigkeit und Dielektrizität) und den physikalischen Eigenschaften (zum Beispiel Phasenverschiebung und Amplitudenreduktion, im Folgenden auch kurz Phase und Amplitude genannt) des elektromagnetischen Signals an der zweiten Position herstellt. Insbesondere ist es möglich, ein solches Modell zu erhalten, indem für verschiedene Bodenarten Messungen der Bodenfeuchte und optional Messungen zumindest einer weiteren Zustandsgröße des Bodens durchgeführt werden und zwar in verschiedenen Zuständen, d.h. bei verschiedenen Werten der Feuchte und optional der Temperatur. Ferner werden für den jeweiligen Zustand die physikalischen Eigenschaften des elektromagnetischen Signals an der zweiten Position ermittelt. Dies erlaubt es bereits, einen rein mathematischen Zusammenhang der zumindest einen elektrischen physikalischen Größe der jeweiligen Bodenart (zum Beispiel elektrische Bodenleitfähigkeit und Dielektrizität) und den physikalischen Eigenschaften (zum Beispiel Phasenverschiebung und Amplitudenreduktion) des Signals herzustellen. Alternativ kann auch eine physikalische Beschreibung dieses Zusammenhangs erstellt werden. Das Modell eines Bodenareals mit mehreren Bodenarten wird aus den zuvor erhaltenen Modellen für die einzelnen Bodenarten zusammengesetzt, d.h. es ergibt sich z. B. für die Phasenverschiebung und die Amplitudenreduktion, dass die durch den ersten Teilbereich hervorgerufene Phasenverschiebung und die Amplitudenreduktion den Zustand an der Grenze zwischen dem ersten und den zweiten Teilbereich beschreibt und der zweite Teilbereich gemäß dem für seine Bodenart gültigen Modell eine weitergehende Phasenverschiebung und Amplitudenreduktion bewirkt. Diese Betrachtung kann auf weitere Teilbereiche entsprechend erweitert werden, d.h. ein auf den zweiten Teilbereich folgender dritter Teilbereich bewirkt gemäß dem für seine Bodenart gültigen Modell eine weitergehende Phasenverschiebung und Amplitudenreduktion.

Gemäß den Ansprüchen wird das Modell an die an der zweiten Position ermittelten Werte der zumindest zwei physikalischen Eigenschaften angepasst. Bei der Anpassung werden Werte der zumindest einen elektrischen physikalischen Größe jedes der Teilbereiche des Bodenareals verändert und somit an die tatsächlichen Verhältnisse angepasst. Dabei wird unter Berücksichtigung von Eigenschaften des jeweiligen Bodens in den Teilbereichen zunächst jeweils ein Startwert der zumindest einen elektrischen physikalischen Größe (insbesondere der Bodenleitfähigkeit und der Dielektrizität) ermittelt. Durch die Anpassung werden in der Regel realitätsnähere Werte der zumindest einen elektrischen physikalischen Größe erhalten. Daraus wiederum wird für die Teilbereiche des Bodenareals jeweils der Feuchtigkeitswert ermittelt. Selbstverständlich unterscheiden sich die mehreren ermittelten Feuchtigkeitswerte in aller

Regel voneinander. Dies liegt bereits daran, dass selbst bei der nicht realistischen Annahme von gleich großen Niederschlagsmengen die einzelnen Bodenarten unterschiedliche Wasser-Speicherkapazitäten haben.

Daher wird vorgeschlagen, dass auf Basis des Modells für die Teilbereiche unter Berücksichtigung von Eigenschaften des jeweiligen Bodens in den Teilbereichen jeweils ein Startwert der zumindest einen elektrischen physikalischen Größe für jeden der Teilbereiche ermittelt wird. Ferner wird das Modell unter Veränderung der Startwerte an die ermittelten Werte des an der zweiten Position empfangenen elektromagnetischen Signals angepasst. Danach werden die Feuchtigkeitswerte der Teilbereiche des Bodenareals basierend auf dem angepassten Modell ermittelt.

Vorzugsweise erfolgt die Anpassung des Modells iterativ durch wiederholte Veränderung der Werte der zumindest einen elektrischen physikalischen Größe. Insbesondere kann die Iteration beendet werden, wenn ein Abbruchkriterium erfüllt ist. Das Abbruchkriterium kann insbesondere darin bestehen, dass bei einem weiteren Iterationsschritt keine wesentlichen Änderungen an den Werten der zumindest einen elektrischen physikalischen Größe mehr auftreten. Wesentliche Änderungen können zum Beispiel so definiert sein, dass sie eine Mindest-Differenzwert dem Betrag nach überschreiten. Es gibt jedoch auch andere Abbruchkriterien, die grundsätzlich auf dem Gebiet der Optimierungsrechnung bekannt sind. Zum Beispiel kann der Gradient (welcher auch als Ableitung der entsprechenden mathematischen Änderungsfunktion bezeichnet werden kann) der Änderungen der zumindest einen elektrischen physikalischen Größe über mehrere Iterationsschritte hinweg ermittelt werden und kann als Abbruchkriterium festgelegt sein, dass die Iteration beim Unterschreiten eines vorgegebenen Betrages des Gradienten beendet wird. Da das Abbruchkriterium einer bestimmten Güte der Anpassung entspricht, kann es auch als Gütekriterium bezeichnet werden.

Die genannten Eigenschaften des jeweiligen Bodens in den Teilbereichen, welche in das Modell eingehen, können insbesondere zumindest eine zustandsunabhängige Eigenschaft und zumindest eine zustandsabhängige Eigenschaft aufweisen. Zustandsunabhängige Eigenschaften sind zum Beispiel Eigenschaften, die sich aus einer Beschreibung der Bodenart in dem Teilbereich ergeben. Ein Beispiel dafür ist die Speicherfähigkeit des Bodens für Wasser. Beispiele für zustandsunabhängige Eigenschaften sind zum Beispiel die Temperatur und auch die Bodenfeuchte. Es ist daher möglich, dass das Modell auch unmittelbar die Bodenfeuchte enthält. Durch Anpassung des Modells in der beschriebenen Weise kann daher unmittelbar für alle Teilbereiche des Bodenareals ein Feuchtigkeitswert erhalten werden.

Ein Ausführungsbeispiel für eine Vorgehensweise zur Bestimmung der Bodenfeuchtigkeit in einem Bodenareale mit einer Mehrzahl von Teilbereichen ist daher: In einem ersten Schritt findet eine Aufteilung des Bodenareals in die Teilbereiche statt, wobei die Aufteilung insbesondere auf Basis zumindest einer für das Bodenareal verfügbaren Bodenkarte stattfindet. In einem zweiten Schritt wird das Modell des Bodenareals aufgestellt. Dabei werden Modellparameter ermittelt, welche die Eigenschaften der Böden in den Teilbereichen beschreiben. Ferner geht die Geometrie der Teilbereiche in das Bodenmodell ein, abhängig von der Lage der Grenzen zwischen den Teilbereichen.

In einem dritten Schritt werden Startwerte für die elektrischen physikalischen Eigenschaften der Böden (d. h. des jeweiligen Bodens) in den Teilbereichen ermittelt. Insbesondere diese elektrischen physikalischen Eigenschaften bestimmen den Einfluss des Bodens auf das an der zweiten Position empfangene elektromagnetische Signal.

Separat von den bisher genannten Schritten werden die Werte der zumindest einen physikalischen Eigenschaft (insbesondere Phase und Amplitude) an der zweiten Position (d. h. am Ort der Empfangseinrichtung) des elektromagnetischen Signals ermittelt oder zum Beispiel aus einem Datenspeicher ausgelesen.

In einem vierten Schritt wird das Modell so angepasst, insbesondere durch Variation von Parametern des Modells, dass die durch das Modell definierten Werte (d. h. die modellierten Werte, die zunächst von den aus der Messung des Signals resultierenden Werten abweichen) der physikalischen Eigenschaft(en) des elektromagnetischen Signals an die aus der Messung ermittelten Werte angenähert werden und idealerweise mit diesen übereinstimmen.

In einem fünften Schritt wird für zumindest einen Teilbereich des Bodenareals auf Basis des angepassten Modells der Feuchtigkeitswert ermittelt.

Die Sendeeinrichtung kann sich zum Teil oder kann sich vollständig an oder in oder bei der ersten Position befinden. Ein Sendeinstrument der Sendeeinrichtung (zum Beispiel eine Sendeantenne) kann sich an oder in oder bei der ersten Position befinden. "Bei" kann zum Beispiel bedeuten, dass ein Abstand nicht mehr als 10 Meter oder 5 Meter oder 1 Meter beträgt. Es ist möglich, dass Teile der Sendeeinrichtung entfernt angeordnet sind, beispielsweise kann an oder in oder bei der ersten Position eine Sendeantenne angeordnet sein, weitere Teile der Sendeeinrichtung können entfernt angeordnet sein.

Die Empfangseinrichtung kann sich zum Teil oder kann sich vollständig an oder in oder bei der zweiten Position befinden. Ein Empfangsinstrument der Empfangseinrichtung (zum Beispiel eine Empfangsantenne) kann sich an oder in oder bei der ersten Position befinden. "Bei" kann zum Beispiel bedeuten, dass ein Abstand nicht mehr als 10 Meter oder 5 Meter oder 1 Meter beträgt. Es ist möglich, dass Teile der Empfangseinrichtung entfernt angeordnet sind, beispielsweise kann an oder in oder bei der zweiten Position eine Empfangsantenne angeordnet sein, weitere Teile der Empfangseinrichtung können entfernt angeordnet sein.

Das Senden an der ersten Position kann insbesondere zum Beispiel ein Senden und/oder Abstrahlen und/oder Absenden bedeuten, und zwar zum Beispiel an und/oder von und/oder ausgehend von und/oder beginnend an der ersten Position.

Das Ermitteln des Wertes der physikalischen Eigenschaft des elektromagnetischen Signals kann sich insbesondere auf das empfangene elektromagnetische Signal beziehen, also das elektromagnetische Signal in einem Zustand, wie es von der Empfangseinrichtung empfangen wird, nachdem es das Bodenareal durchlaufen hat.

Der Feuchtigkeitswert kann insbesondere eine quantitative Angabe sein (zum Beispiel der volumetrische Wasseranteil). Es kann sich allgemeiner formuliert um einen Wert der Bodenfeuchte handeln, zum Beispiel ausgedrückt in Prozent der nutzbaren Feldkapazität. Der Feuchtigkeitswert kann alternativ zum Beispiel ein Wasservolumen pro Volumeneinheit des Bodens beschreiben, zum Beispiel ein Wasservolumen in Litern oder Kubikmetern pro Kubikmeter des Bodenareals. Die Angabe kann in Volumenprozent erfolgen. Der Feuchtigkeitswert kann auch eine andere im Stand der Technik bekannte Angabe für die Feuchtigkeit sein. Es ist nicht ausgeschlossen, dass der Feuchtigkeitswert auch eine qualitative Angabe sein kann, zum Beispiel eine Angabe aus der Menge "nass, feucht, frisch, dürr, trocken" wie in der Bodenkunde üblich.

Die erste Position kann wie bereits erläutert eine Position auf dem Boden, insbesondere auf dem Erdboden oder auf der Erdoberfläche sein. Die Position kann an Land befindlich sein. Es ist nicht ausgeschlossen, dass die Position sich alternativ über, unter oder an einer Wasseroberfläche oder einer Eisoberfläche befindet, zum Beispiel über, auf oder innerhalb (zum Beispiel nahe über, nahe an, auf oder nahe unterhalb der Wasseroberfläche oder der Eisoberfläche) oder eines Sees oder eines Meeres. Die erste Position kann sich dann zum Beispiel auf einer schwimmfähigen Vorrichtung, zum Beispiel einer Boje, einem Schwimmkörper, einem Boot oder einem Schiff befinden. Die erste Position kann im Verlauf mehrerer Anwendungen des Verfahrens veränderlich sein. Mehrere Sendeeinrichtungen können verwendet werden, um unterschiedliche Bodenareale zu untersuchen.

Die zweite Position kann wie bereits erläutert ebenfalls eine Position auf dem Boden, insbesondere auf dem Erdboden oder auf der Erdoberfläche, oder über dem Boden (z. B. über der Erdoberfläche) sein. Die Position kann an Land befindlich sein. Es ist nicht ausgeschlossen, dass die Position sich alternativ über, unter oder an einer Wasseroberfläche oder einer Eisoberfläche befindet, zum Beispiel über, auf oder innerhalb (zum Beispiel nahe über, nahe an, auf oder nahe unterhalb der Wasseroberfläche oder der Eisoberfläche) eines Sees oder eines Meeres. Die zweite Position kann sich dann zum Beispiel auf einer schwimmfähigen Vorrichtung, zum Beispiel einer Boje, einem Schwimmkörper, einem Boot oder einem Schiff befinden. Die zweite Position kann im Verlauf mehrerer Anwendungen des Verfahrens veränderlich sein. Mehrere Empfangseinrichtungen können verwendet werden, um unterschiedliche Bodenareale zu untersuchen.

Das Bodenareal befindet sich zwischen der ersten Position und der zweiten Position. Insbesondere kann es sich zwischen der ersten Position und der zweiten Position befinden, wenn es im Grundriss betrachtet wird. Der Begriff "zwischen" kann insbesondere derart zu verstehen sein, dass sich das Bodenareal in einer Draufsicht - also von oben betrachtet - zwischen der ersten Position und der zweiten Position befinden kann. Das heißt, dass zum Beispiel aus der Perspektive eines Satelliten oder eines Flugzeugs, dass sich mittig oberhalb des Bodenareals befindet, das Bodenareal sich zwischen der ersten Position der zweiten Position befindet. Das Bodenareal kann sich zwischen der ersten Position und der zweiten Position auf dem Großkreis und/oder entlang des Großkreises befinden, der durch die erste Position und die zweite Position definiert wird. Das Bodenareal kann sich insbesondere in Querrichtung ausgehend von dem Großkreis zu beiden Querseiten erstrecken.

In Bezug auf die vertikale Lage kann sich das Bodenareal von einer gedachten Linie, die sich zwischen der ersten Position und der zweiten Position erstreckt, vertikal entfernen. Das heißt, das Bodenareal kann in Bezug auf die gedachte Linie höhere oder tiefere Abschnitte, zum Beispiel verursacht durch Hügel oder Täler oder die Erdkrümmung, aufweisen.

Gemäß der oben beschriebenen vorgeschlagenen Lösung weist das Bodenareal eine Mehrzahl von Teilbereichen auf, die bezüglich einer entlang der Bodenoberfläche des Bodenareals verlaufenden direkten Verbindungslinie hintereinander liegen. Die direkte Verbindungslinie kann in einer senkrechten Draufsicht auf das Bodenareal direkt von der ersten Position zu der zweiten Position verlaufen. Da die Bodenoberfläche aber gemäß der Erdkrümmung gekrümmt verläuft, verläuft auch die direkte Verbindungslinie jedenfalls nicht geradlinig, bzw. lediglich in einer senkrechten Draufsicht geradlinig. Insbesondere verläuft die direkte Verbindungslinie entlang eines Großkreises der Erde oder des anderen Planeten von der ersten Position zur zweiten Position. Bevorzugt wird ferner, dass die direkte Verbindungslinie auch den Höhenunterschieden der Oberfläche der Teilbereiche folgt. Dies ist aber insbesondere von der Art des Modells abhängig, nämlich wie das Modell das Bodenareal geometrisch beschreibt. Es ist insbesondere auch möglich, dass das Bodenmodell keine Höhenunterschiede berücksichtigt oder diese angenähert berücksichtigt.

Allgemein kann eine Ermittlung des Feuchtigkeitswerts des Bodenareals bedeuten, dass der Feuchtigkeitswert eines Teilbereichs des Bodenareals ermittelt werden soll. Ein Teilbereich, der mithilfe des erfindungsgemäßen Verfahrens untersucht werden soll, muss nicht direkt mit der ersten oder der zweiten Position räumlich verbunden sein oder an sie angrenzen. Dies kann insbesondere der Fall sein, wenn sich zwischen der ersten und der zweiten Position Land- und Wasserabschnitte befinden und alleine ein oder mehrere Landabschnitte untersucht werden soll/sollen. Der zu untersuchende Teilbereich kann in der Längsausdehnung kleiner sein als die Distanz zwischen der ersten Position und der zweiten Position.

Das Bodenareal kann in der Richtung der Distanz zwischen der ersten und der zweiten Position, also in Längsausdehnung, zum Beispiel Längsausdehnungen in der Größenordnung von Dekametern, Hektometern, Kilometern, Vielfachen von 10 Kilometern oder Vielfachen von 100 Kilometern oder Vielfachen von 1000 Kilometern haben.

Eine Längsausdehnung des Bodenareals kann also zum Beispiel
- mindestens 10 Meter betragen oder in der Größenordnung von mehreren (z. B. 1-10) Dekametern liegen oder
- mindestens 100 Meter betragen oder in der Größenordnung von mehreren (z. B. 1-10) Hektometern liegen oder
- mindestens 1 km betragen oder in der Größenordnung von mehreren (z. B. 1-10) Kilometern liegen oder
- mindestens 10 km betragen oder in der Größenordnung von mehreren (z. B. 1-10) 10 km liegen oder
- mindestens 100 km betragen oder in der Größenordnung von mehreren (z. B. 1-10) 100 km liegen oder
mindestens 1000 km betragen oder in der Größenordnung von mehreren (z. B. 1-10) 1000 km liegen.

In Querausdehnung, also quer zur Richtung der Distanz zwischen der ersten und der zweiten Position, kann das Bodenareal zum Beispiel Ausdehnungen in der Größenordnung von Metern, Dekametern, Hektometern oder Kilometern oder 10 Kilometern haben.

Eine Querausdehnung des Bodenareals kann also zum Beispiel
- mindestens 10 Meter betragen oder in der Größenordnung von mehreren (z. B. 1-10) Dekametern liegen oder
- mindestens 100 Meter betragen oder in der Größenordnung von mehreren (z. B. 1-10) Hektometern liegen oder
- mindestens 1 km betragen oder in der Größenordnung von mehreren (z. B. 1-10) Kilometern liegen oder
mindestens 10 km betragen oder in der Größenordnung von mehreren (z. B. 1-10) 10 km liegen.

Auch Größenordnungen darüber (mindestens 100 km oder in der Größenordnung von mehreren (z. B. 1-10) 100 km sind für die Querausdehnung nicht ausgeschlossen.

Die Querausdehnung kann auch anders festgelegt oder definiert werden, zum Beispiel anhand von Erfahrungswerten oder unter Berücksichtigung von Bodenkarten, insbesondere solchen Bodenkarten, die Verteilungen der verschiedenen Bodenarten zwischen der ersten Position und der zweiten Position aufzeigen.

Der Feuchtigkeitswert kann ein durchschnittlicher oder repräsentativer Feuchtigkeitswert des Bodenareals sein. Die Querausdehnung des Bodenareals kann per Definition derart festgelegt werden, dass sie breiter ist, als der Bereich, in dem tatsächlich eine (messbare und/oder nicht völlig unerhebliche) Einflussnahme auf das elektromagnetische Signal durch den Boden auftritt.

Eine real zutreffende Querausdehnung des Bodenareals, das mit dem erfindungsgemäßen Verfahren tomographisch untersucht wird, könnte zum Beispiel experimentell ermittelt werden. So kann eine Definition der Querausdehnung des Bodenareals vorgenommen werden, dass sie dem Bereich entspricht, in dem tatsächlich eine (messbare und/oder nicht unerhebliche) Einflussnahme auf das elektromagnetische Signal durch den Boden auftritt. Zum Beispiel könnten mithilfe des erfindungsgemäßen Verfahrens ermittelte Feuchtigkeitswerte mit anders ermittelten Feuchtigkeitswerten des Bodenareals verglichen werden, um so eine Querausdehnung des Bodenareals durch Abgleich zu bestimmen. So kann eine hohe Aussagegüte des mit dem erfindungsgemäßen Verfahren zu erhaltenden Feuchtigkeitswerts realisiert werden.

Wie bereits erwähnt kann die Querausdehnung auch ausgehend von dem Großkreis zwischen der ersten Position und der zweiten Position sich seitlich zu beiden Seiten jeweils um eine Fresnel-Halbwellen-Zone lang erstrecken (Gesamtbreite zwei Fresnel-Halbwellen-Zonen). Insoweit kann auch eine Definition der Querausdehnung des Bodenareals derart vorgenommen werden, dass sie dem Bereich entspricht, in dem tatsächlich eine (messbare und/oder nicht unerhebliche) Einflussnahme auf das elektromagnetische Signal durch den Boden auftritt.

Die Querausdehnung muss nicht genau bestimmbar sein oder bestimmt werden. Der Wert der physikalischen Eigenschaft kann ein durchschnittlicher oder repräsentativer Wert des Bodenareals sein wie bereits erläutert. Die Querausdehnung des Bodenareals kann ein definierter Wert sein. Die Querausdehnung des Bodenareals kann zudem mithilfe weiterer Bodendaten ermittelbar sein. Wenn zum Beispiel bekannt ist, dass sich in einer bekannten Breite zwischen der ersten Position und der zweiten Position Boden einer einheitlichen Bodenart erstreckt, kann das Bodenareal in der Querausdehnung dieser bekannten Breite entsprechen. Die Form des Bodenareals kann, muss aber nicht ein Rechteck sein.

Die Sendeeinrichtung kann insbesondere einen Sender aufweisen, also eine Einrichtung zur Erzeugung und Abstrahlung von elektromagnetischen Wellen. Insbesondere kann sie einen Oszillator, einen Verstärker und/oder eine Sendeantenne aufweisen. Die Sendeeinrichtung kann eine Recheneinheit aufweisen oder mit einer Recheneinheit verbunden sein. Die Sendeeinrichtung kann mit dem Boden elektrisch verbunden sein. Insbesondere kann die Sendeeinrichtung eine Antenne aufweisen, die mit dem Boden elektrisch verbunden sein kann. Ein Teil der Antenne kann sich in den Boden erstrecken. Alternativ oder zusätzlich kann die Sendeeinrichtung mobil, also in konstruktiver Hinsicht ab- und wieder aufbaubar oder transportabel gestaltet sein und/oder sich auf einer mobilen Vorrichtung (zum Beispiel Fahrzeug, Boot, Schiff, allgemein: boden- oder wassergestützter Träger) befinden.

Die Sendeeinrichtung kann eine Anlage zur Positionsbestimmung und/oder zur Zeitbestimmung aufweisen, zum Beispiel einen Empfänger eines globalen Navigationssatellitensystems oder eines terrestrischen Navigationssystems oder eines sonstigen Navigationssystems. Die Anlage zur Positionsbestimmung und zur Zeitbestimmung kann bei der Ermittlung des Wertes der physikalischen Eigenschaft des elektromagnetischen Signals (insbesondere eines Phasenwertes oder eines Wertes einer Laufzeit) genutzt werden. Alternativ zu einer Anlage zur Positionsbestimmung kann eine Ortsinformation in Bezug auf die Sendeeinrichtung auch auf andere Weise ermittelt werden, zum Beispiel mithilfe geodätischer Einmessung. Eine Zeitbestimmung kann zum Beispiel mithilfe des Signals eines Mobilfunkanbieters erfolgen.

Die Empfangseinrichtung kann insbesondere einen Empfänger aufweisen. Insbesondere kann der Empfänger eine Antenne, einen Filter und/oder einen Verstärker aufweisen. Die Empfangseinrichtung kann zum Beispiel eine Dipolantenne und/oder eine magnetische Antenne und/oder eine Ferritantenne aufweisen. Die Empfangseinrichtung kann eine Recheneinheit aufweisen oder mit einer Recheneinheit verbunden sein. Die Empfangseinrichtung kann mit dem Boden elektrisch verbunden sein. Insbesondere kann die Empfangseinrichtung eine Antenne aufweisen, die mit dem Boden elektrisch verbunden sein kann. Ein Teil der Antenne kann sich in den Boden erstrecken. Alternativ oder zusätzlich kann die Empfangseinrichtung mobil, also in konstruktiver Hinsicht ab- und wieder aufbaubar oder transportabel gestaltet sein und/oder sich auf einer mobilen Vorrichtung (zum Beispiel Fahrzeug, Boot, Schiff) befinden. Die Empfangseinrichtung kann sich auch auf einem flugfähigen Gerät oder einem flugfähigen Träger oder einem Flugzeug oder einem Hubschrauber oder einem Leichtflugzeug oder einem Segelflugzeug oder einer flugfähigen Drohne oder an einem Ballon oder einem Drachen befinden, so sind Positionswechsel besonders einfach möglich oder sind auch Empfangsvorgänge aus der Luft, zum Beispiel und bevorzugt in Bodennähe, möglich. Eine solche Ausgestaltung ermöglicht insbesondere Untersuchungen von schwer zugänglichen Bodenarealen.

Die Empfangseinrichtung kann eine Anlage zur Positionsbestimmung und/oder zur Zeitbestimmung aufweisen, zum Beispiel einen Empfänger eines globalen Navigationssatellitensystems oder eines terrestrischen Navigationssystems oder eines sonstigen Navigationssystems. Die Anlage zur Positionsbestimmung und zur Zeitbestimmung kann bei der Ermittlung des Wertes der physikalischen Eigenschaft des elektromagnetischen Signals (insbesondere eines Phasenwertes oder eines Wertes einer Laufzeit) genutzt werden. Alternativ zu einer Anlage zur Positionsbestimmung kann eine Ortsinformation in Bezug auf die Empfangseinrichtung auch auf andere Weise ermittelt werden, zum Beispiel mithilfe geodätischer Einmessung. Eine Zeitbestimmung kann zum Beispiel mithilfe des Signals eines Mobilfunkanbieters erfolgen.

Das elektromagnetische Signal kann, im einfachsten Fall, eine Sinusschwingung aufweisen. Das elektromagnetische Signal kann auch mehrere Schwingungen in unterschiedlichen Frequenzen, die überlagert sind, aufweisen. Das Signal kann das Signal mehrere, zum Beispiel zwei, Carrier-Signale (z. B. Sinusschwingungen) aufweisen und einen Datenkanal. Das elektromagnetische Signal kann Informationen enthalten. Das elektromagnetische Signal oder eine Signalkomponente kann moduliert sein und dadurch Träger von Informationen sein. Zum Beispiel kann ein Identifikationsmerkmal enthalten sein, das zum Beispiel eine Identifikation der Sendeeinrichtung ermöglicht. Das elektromagnetische Signal kann andere Informationen enthalten, zum Beispiel Informationen über die Position der Sendeeinrichtung, über Fehlerkorrekturen und/oder über einen Zeitpunkt. Der Zeitpunkt kann zum Beispiel den Sendezeitpunkt des elektromagnetischen Signals, einer definierten Eigenschaft des elektromagnetischen Signals oder einer bestimmten Information des elektromagnetischen Signals kennzeichnen. So können anhand des empfangenen elektromagnetischen Signals der Sendezeitpunkt und ein Ausbreitungspfad des elektromagnetischen Signals ermittelt werden. Zum Beispiel kann eine solche Ermittlung automatisch durch die Empfangseinrichtung, insbesondere eine Recheneinheit der Empfangseinrichtung, erfolgen.

Zum Beispiel kann ein Nulldurchgang der Phase des elektromagnetischen Signals oder einer oder mehrerer Signalkomponenten des elektromagnetischen Signals zu jeder vollen Sekunde an der ersten Position erfolgen. An der zweiten Position wird das elektromagnetische Signal zeitlich verzögert empfangen. Dies führt dazu, dass zur vollen Sekunde jeweils an der zweiten Position (z. B. an der Empfangseinrichtung) das elektromagnetische Signal bzw. die Signalkomponente/-n des elektromagnetischen Signals eine Phasenverschiebung bzw. Phasenverschiebungen aufweist/aufweisen, die von der vorliegenden Laufzeit und der (jeweils) vorliegenden Frequenz abhängen kann/können.

An der Empfangseinrichtung kann eine Abweichung/Phasenverschiebung eines Nulldurchgangs zu einer vollen Sekunde (oder ein Durchschnittswert solcher Abweichungen/Phasenverschiebungen) als Phasenwert der Wert der physikalischen Eigenschaft des elektromagnetischen Signals sein. Ein solcher Phasenwert kann auch eine oder mehrere Signalkomponenten des elektromagnetischen Signals betreffen.

Die Abweichung als Zeiteinheit (oder ein Durchschnittswert solcher Abweichungen) kann als Zeitangabe alternativ der Wert der physikalischen Eigenschaft des elektromagnetischen Signals sein.

Eine Identifikation oder zumindest eine Unterscheidbarkeit von Sendeeinrichtungen ist in einem einfachen Fall zum Beispiel mithilfe individueller Frequenzen möglich. So ist es möglich, dass mehrere Sendeeinrichtungen mit jeweils unterschiedlichen Frequenzen in einem zu untersuchenden Bodengebiet angeordnet sind, zum Beispiel jeweils in einem vorgegebenen Abstand voneinander angeordnet. Die elektromagnetischen Signale der Sendeeinrichtungen können zum Beispiel aufgezeichnet werden und zu einem tomographischen Bild des Bodengebiets zusammengestellt werden.

Alternativ können zur Identifikation von verschiedenen Sendeeinrichtungen die verschiedenen Sendeeinrichtungen nach einem vordefinierten Schema oder nach einem veränderlichen, zum Beispiel sich adaptiv an sich ändernde Bedingungen anpassenden Schema, zum Beispiel nacheinander auf gleichen oder teilweise überlappenden Frequenzen, jeweils ein elektromagnetisches Signal senden. Eine solche Bedingung könnte zum Beispiel ein Hinzufügen oder ein Entfernen oder ein Ausfall einer Sendeeinrichtung sein.

Auch eine Kombination aus Sendevorgängen auf verschiedenen Frequenzen und zu unterschiedlichen Zeiten zur Identifikation verschiedener Sendeeinrichtungen ist möglich.

Der Empfangseinrichtung kann ein Schema und/oder können Frequenzen der Sendeeinrichtungen bekannt sein, durch das (jeweilige) elektromagnetische Signal mitgeteilt werden oder mit Hilfe eines weiteren Datenkanals der Empfangseinrichtung zur Verfügung gestellt werden.

Grundsätzlich ist sinnvoll, dass elektromagnetische Signale verschiedener Sendeeinrichtungen ein oder mehrere spezifische Merkmale aufweisen, die der Empfangseinrichtung ermöglichen, zweifelsfrei das jeweils empfangene elektromagnetische Signal einer jeweiligen Sendeeinrichtung zuzuordnen. Mögliche Ausgestaltungen solcher spezifischen Merkmale, die im jeweiligen elektromagnetischen Signal enthalten sein können, können zum Beispiel sein: eine Sendeeinrichtungs-ID, eine oder mehrere definierte Frequenzen, ein definierter Zeitslot der Sendeeinrichtung, ein modulierter oder gepulster Signalanteil mit definierten Eigenschaften, eine Frequenzrampe.

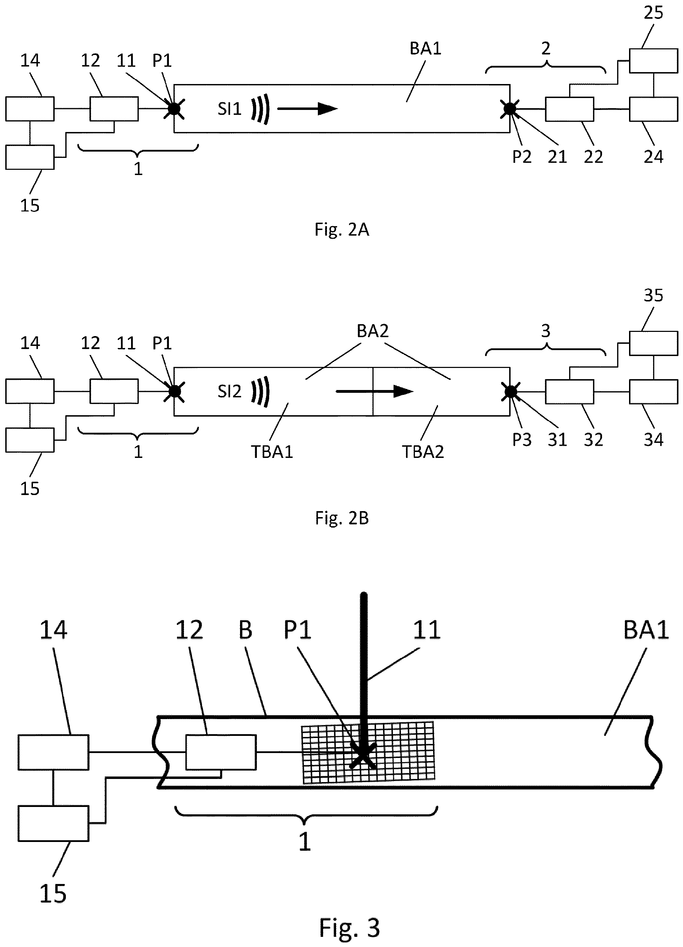

Abhängig von ihrer Frequenz haben elektromagnetische Wellen unterschiedliche Ausbreitungsverhalten als Bodenwellen. Allgemein gilt, dass elektromagnetische Wellen mit vergleichsweise langen Wellenlängen (zum Beispiel Mittelwelle oder Langwelle) sich weiter im Boden als Bodenwellen ausbreiten (das heißt mit der Entfernung weniger gedämpft werden) als elektromagnetische Wellen mit vergleichsweise kurzen Wellenlängen. Jene werden mit der Entfernung stärker gedämpft. Für das elektromagnetische Signal sind bei größeren Distanzen (zum Beispiel im Bereich oberhalb von 100 km oder oberhalb von 1000 km) daher tendenziell größere Wellenlängen (Mittelwelle, Langwelle) empfehlenswert. Für kleinere Distanzen (zum Beispiel unterhalb von 100 km oder unterhalb von 10 km) sind, weil hierdurch eine exaktere Bestimmung des Wertes der physikalischen Eigenschaft ermöglicht wird (z. B. Phasenwert oder Wert einer Laufzeit), tendenziell kürzere Wellenlängen vorteilhaft, sofern die Dämpfung nicht zu hoch ist. Daten hierzu (zum Beispiel Dämpfung von Bodenwellen über der Distanz mit der Frequenz als Parameter) finden sich zum Beispiel in Angulo et al., 2014, "Handbook on Ground Wave Propagation", International Telecommunication Union (ITU), Seite 9, Fig. 3.

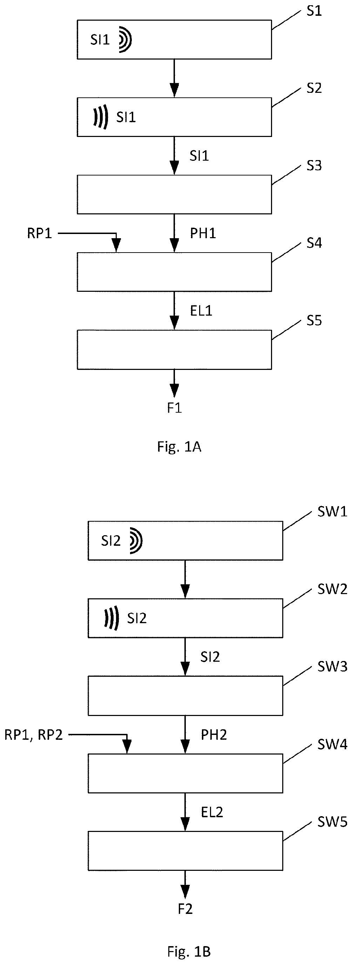

Im Rahmen der vorliegenden Erfindung kann es eine Voraussetzung sein, dass das elektromagnetische Signal sich als Bodenwelle verbreitet, das heißt, dass das Bodenareal mindestens teilweise in einem Tiefenbereich nahe der Oberfläche durchlaufen wird. Es kann eine Voraussetzung sein, dass das elektromagnetische Signal als Bodenwelle empfangen wird. Der Tiefenbereich nahe der Oberfläche kann zum Beispiel in der Größenordnung von Zentimetern, Dezimetern oder Metern liegen (vgl. zum Beispiel Angulo et al., 2014, "Handbook on Ground Wave Propagation", International Telecommunication Union (ITU), Seite 11, Abschnitt 6, "Ground conductivity" und Recommendation P527-5 (08/2019) "Electrical characteristics of the surface of the Earth" der International Telecommunication Union (ITU), Fig. 1.

Dem Fachmann ist bekannt, bei welcher Frequenz und bei welcher senderseitigen elektrischen Leistung eine Bodenwelle in gewünschter Eindringtiefe über eine gewünschte Distanz (die zum Beispiel die Längsausdehnung des Bodenareals sein kann) erhalten wird. Der Fachmann kann daher, je nach gewünschtem Anwendungsfall, eine sinnvolle Frequenz und eine sinnvolle elektrische Leistung für das elektromagnetische Signal wählen (vgl. z. B. Recommendation P527-3 (03/1992) "Electrical characteristics of the surface of the Earth" der International Telecommunication Union (ITU, insbesondere Fig. 3). Eine elektrische Welle, die als Bodenwelle abgestrahlt wird, kann auch Anteile besitzen, die nicht als Bodenwelle, sondern zum Beispiel als Raumwelle abgestrahlt werden. Ein Raumwellenanteil kann daher beim erfindungsgemäßen Verfahren zusätzlich auftreten, ist jedoch für die Ermittlung des Feuchtigkeitswerts ohne Relevanz.

Der Wert der physikalischen Eigenschaft des elektromagnetischen Signals kann sich insbesondere auf das empfangene elektromagnetische Signal beziehen, also einen empfangsseitigen Zustand des elektromagnetischen Signals. Der Wert der physikalischen Eigenschaft kann insbesondere ein Phasenwert des elektromagnetischen Signals zu einem vordefinierten Zeitpunkt (zum Beispiel nach Ablauf einer vordefinierten Zeit seit dem Absenden des elektromagnetischen Signals durch die Sendeeinrichtung) sein. Der Wert der physikalischen Eigenschaft kann insbesondere auch ein Wert einer Laufzeit des elektromagnetischen Signals sein. Die Ermittlung des Werts der physikalischen Eigenschaft kann zum Beispiel messtechnisch und/oder rechnerisch und/oder mithilfe einer Recheneinheit erfolgen. Eine solche Recheneinheit (wie auch andere Recheneinheiten, die im Text erwähnt werden) kann einen Prozessor und einen Speicher aufweisen. Die Recheneinheit kann datentechnisch (zum Beispiel über eine kabelgebundene oder über eine drahtlose Verbindung) mit der Empfangseinrichtung verbunden sein oder in ihr integriert sein.

Bei dem Ermitteln des Feuchtigkeitswerts des Bodenareals kann zusätzlich ein Referenzwert genutzt werden. Der Referenzwert kann dieselbe physikalische Eigenschaft des elektromagnetischen Signals betreffen. Es kann sich also ebenfalls insbesondere um einen Phasenwert oder um einen Wert einer Laufzeit handeln. Der Referenzwert kann aus einer früheren Ermittlung mithilfe des vorgestellten Verfahrens stammen, wobei bei dieser früheren Ermittlung kein eigener Referenzwert vorhanden gewesen sein muss. So kann ein Vergleich von Feuchtigkeitswerten im zeitlichen Verlauf stattfinden. Ein Unterschied zwischen dem Referenzwert und dem Wert der physikalischen Eigenschaft kann daher eine Veränderung des Feuchtigkeitswerts des Bodenareals darstellen. Der Referenzwert kann auch ein theoretisch oder experimentell ermittelter Wert sein, ermittelt zum Beispiel unter Hinzuziehung von Zusatzinformationen (zum Beispiel Bodenkarten, bekannte Bodenparameter). Der Referenzwert kann ein theoretisch oder experimentell oder mithilfe einer früheren Anwendung des vorgestellten Verfahrens ermittelter Erwartungswert der physikalischen Eigenschaft sein, der bei einem bekannten Feuchtigkeitswert des Bodenareals auftritt. Es kann zum Beispiel ein Vergleich des Wertes der physikalischen Eigenschaft mit dem Referenzwert bei der Bestimmung des Feuchtigkeitswerts des Bodenareals durchgeführt werden.

Mithilfe des Wertes der physikalischen Eigenschaft des elektromagnetischen Signals kann ein Wert einer elektrischen physikalischen Größe des Bodenareals ermittelt werden. Zum Beispiel sind Zusammenhänge bekannt, wie mithilfe eines bekannten Werts einer elektrischen Leitfähigkeit, einer Impedanz oder einer Permittivität ein zu erwartender Phasenwert oder Wert einer Signallaufzeit bestimmt werden kann. Derartige Zusammenhänge können, zum Beispiel numerisch, rückwärts - also bei ermitteltem Phasenwert - ausgeführt werden, um auf den gewünschten Wert der elektrischen physikalischen Größe des Bodenareals zu schließen. Bekannte Bodenparameter (zum Beispiel Materialien oder Zusammensetzungen) oder Bodenkarten können hierbei berücksichtigt werden.

Anders ausgedrückt basieren diese Zusammenhänge auf einem Modell des Bodenareals. Bereits da es sich in dem hier beschriebenen Fall um Zusammenhänge zwischen der zumindest einen physikalischen Eigenschaft des elektromagnetischen Signals und der zumindest einen elektrischen physikalischen Größe des Bodenareals handelt, kann von einem physikalischen Modell gesprochen werden. Es ist dabei möglich, dass auch der Zusammenhang durch physikalische Beziehungen ausgedrückt wird. Alternativ oder zusätzlich kann der Zusammenhang durch nicht-physikalische, rein mathematische Beziehungen in dem Modell beschrieben werden. Von dem Modell selbst ist die Implementierung des Modells zu unterscheiden. Insbesondere kann auch ein Modell, welches die genannten physikalischen Beziehungen enthält, durch rein numerische Verfahren implementiert werden. Dies kann insbesondere dann der Fall sein, wenn das Modell zwar zumindest eine Gleichung aufweist, welche den physikalischen Zusammenhang zwischen zumindest einer physikalischen Eigenschaft des elektromagnetischen Signals und zumindest einer elektrischen physikalischen Größe des Bodenareals beschreibt, diese zumindest eine Gleichung aber keine analytische Lösung hat.

In der Veröffentlichung Pressey et al., 1952, "The Measurement of the Phase Velocity of Ground-Wave Propagation at low Frequencies over a Land Path", Proceedings of the IEE - Part III: Radio and Communication Engineering Volume 100, Issue 64, 1953 wird beispielsweise beschrieben, dass eine Änderung eines Landschaftstyps in einem betrachteten Areal eine Änderung einer elektrischen Leitfähigkeit des Bodens in dem betrachteten Areal und eine Änderung eines Phasenwerts einer Bodenwelle, die durch den Boden in dem betrachteten Areal propagiert, bewirkt. Im Rahmen der vorliegenden Erfindung wurde festgestellt, dass sich ähnliche Änderungen bei einer Änderung eines Feuchtigkeitsgehalts ergeben. Eine umgekehrte Betrachtung ermöglicht es, von einem gemessenen Phasenwert auf eine Änderung der elektrischen Leitfähigkeit zu schließen. Weitere Angaben und Berechnungsvorschriften finden sich zum Beispiel auch in Wait, J. R., et al., 1998, "The Ancient and Modern History of EM Ground-Wave-Propagation", IEEE Antennas and Propagation Magazine, Vol. 40, No. 5, October 1998.

Ferner beschreibt die International Telecommunication Union ITU in ihrer Recommendation ITU-R P.527-6 (09/2021) mit dem Titel "Electrical characteristics of the surface of the Earth" insbesondere in Abschnitt 5.2 "Soil" den Zusammenhang zwischen der Dielektrizität (hier der relativen Permittivität) des Bodens, der Frequenz des Signals, der Temperatur, der Bodenzusammensetzung und dem Wassergehalt (hier der dem volumetrischen Wassergehalt). Die Formeln (57) bis (70) können als mathematische Beschreibung des Modells verwendet werden. Wenn die Variablen dieser Gleichungen mit Ausnahme der Feuchtigkeit bekannt sind, kann daraus die Feuchtigkeit ermittelt werden. Die Gleichungen werden hier nicht wiederholt, da sie in der genannten Veröffentlichung in Abschnitt 5.2 enthalten und beschrieben sind. Grundlage für die Beschreibung der Bodeneigenschaften ist die bekannte Modellannahme, dass es die Bodenarten Sand, Schluff, Lehm und Mischungen davon gibt. Die entsprechende Formel (57) für die Massendichte des Bodens findet sich auf Seite 18 der Veröffentlichung. Die weiteren genannten Formeln folgen ab Seite 19. Bei den Gleichungen handelt sich um eine Beschreibung des Modells, die z. B. wie oben beschrieben durch empirische Bestimmung von Modellparametern und Messung von Feuchtigkeit und anderen Zustandsgrößen aufgestellt werden kann.

Eine Permittivität, eine elektrische Leitfähigkeit oder eine Impedanz können mehrmals, bei verschiedenen Frequenzen des elektromagnetischen Signals, ermittelt werden. So kann eine Permittivität, eine elektrische Leitfähigkeit oder eine Impedanz frequenzabhängig für das Bodenareal bestimmt werden.

Der Feuchtigkeitswert des Bodenareals kann mithilfe des Wertes der elektrischen physikalischen Größe des Bodenareals ermittelt werden. Dies kann unter Nutzung einer Recheneinheit, insbesondere eines Computers, erfolgen. Der Wert der elektrischen physikalischen Größe kann insbesondere ein Wert der elektrischen Leitfähigkeit, ein Wert der Impedanz oder eine dielektrische Eigenschaft (insbesondere eine relative Dielektrizitätskonstante) des Bodenareals sein. Es ist auch möglich, dass der Referenzwert (sofern er genutzt wird) ein solcher Wert ist. Es kann zum Beispiel ein Vergleich des Wertes der elektrischen physikalischen Größe mit dem Referenzwert bei der Bestimmung des Feuchtigkeitswerts des Bodenareals durchgeführt werden. Andere Einflussfaktoren wie die Temperatur des Bodenareals oder eines Wassergehalts im Bodenareal oder der Frequenzabhängigkeit von elektrischen Eigenschaften des Wassers können zusätzlich berücksichtigt werden.

Es ist nicht ausgeschlossen, dass der Feuchtigkeitswert des Bodenareals ohne den Zwischenschritt über den Wert der elektrischen physikalischen Größe des Bodenareals ermittelt wird, zum Beispiel über Zusammenhänge, die als Datenbank, Tabelle, Wertesammlung oder Kennlinie oder als Funktion vorliegen. Derartige Zusammenhänge können durch frühere Messungen ermittelt worden sein und direkte Zusammenhänge zwischen dem Wert der physikalischen Eigenschaft des elektromagnetischen Signals und dem Feuchtigkeitswert beschreiben. Die Zusammenhänge können insbesondere Referenzwerte der physikalischen Eigenschaft des elektromagnetischen Signals aufweisen oder berücksichtigen.

Das Bodenareal kann mehrere Bodenbereiche unterschiedlicher Materialien oder Zusammensetzungen aufweisen (zum Beispiel Ton, Sand, Lehm, Erde etc.). Für die Bodenbereiche kann der Wert der elektrischen physikalischen Größe unter Nutzung von Zusatzinformationen (zum Beispiel Bodenkarten, bekannte Bodenparameter) jeweils separat ermittelt werden. Hierbei können auch georeferenzierte Objektklassifikationen oder Landbedeckungsklassifikationen berücksichtigt werden.

Für die Bodenbereiche können alternativ oder zusätzlich separate Feuchtigkeitswerte ermittelt werden, zum Beispiel mittels mehrfacher Anwendung des erfindungsgemäßen Verfahrens an Positionen, zwischen denen die Bodenbereiche liegen.

Eine Veränderung des Wertes der elektrischen physikalischen Größe des Bodenareals lässt auf eine Veränderung des Feuchtigkeitswert des Bodenareals schließen. Derartige Zusammenhänge sind insbesondere für die elektrische Leitfähigkeit, die Dielektrizitätskonstante und die Impedanz bekannt, zum Beispiel in:
- Hippel von, A.R. (1954): "Dielectric Materials and Applications" New York: Technology press of Massachusetts Institute of Technology, S. 438 ff.
- Nelson, S., D. Lindroth, and R. Blake (1989), "Dielectric properties of selected minerals at 1 to 22 GHz", Geophysics, 54(4), S. 1334-1349.
- Ulriksen, C.P.F., 1982. "Application of impulse radar to civil engineering" Ph.D. Thesis: Univ. Lund, Lund, S. 175 ff.
- de Vries, D.A. (1963). "Thermal Properties of Soils", in: van Wijk, W.R. (ed.): "Physics of Plant Environment", Amsterdam: North Holland Publishing Company, S. 210-235. Arenson, L.U., Colgan, W., Marshall, H.-P. (2015): "Physical, Thermal, and Mechanical Properties of Snow, Ice, and Permafrost (Chapter 2.) - Snow and Ice-Related Hazards, Risks, and Disasters", S. 35 - 75. doi: 10.1016/B978-0-12-394849-6.00002-0.

Ein Zusammenhang zwischen dem Wert der elektrischen physikalischen Größe und dem Feuchtigkeitswert kann - zum Beispiel unterteilt nach verschiedenen Bodenbereichen unterschiedlicher Materialien - als Datenbank, Tabelle, Wertesammlung oder Kennlinie oder als funktionaler Zusammenhang vorliegen. Eine solche Datenbank, Tabelle, Wertesammlung oder Kennlinie kann bevorzugt für spezifische Bodenarten spezifische Zusammenhänge zwischen Werten der elektrischen physikalischen Größe und Feuchtigkeitswerten aufweisen. Das Ermitteln des Feuchtigkeitswertes des Bodenareals mithilfe des Wertes der physikalischen Eigenschaft kann mithilfe einer Recheneinrichtung, insbesondere mithilfe eines Computers (insbesondere aufweisend eine CPU und einen Speicher), erfolgen.

Durch mehrfache Anwendung des Verfahrens können mehrere Bodenareale hinsichtlich ihrer Feuchtigkeitswerte untersucht werden. Ermittelte Feuchtigkeitswerte können zum Beispiel auf einer Karte oder als zweidimensionales Bild dargestellt werden und an den entsprechenden Bodenarealen dargestellt werden. Auch eine Darstellung als Punktwolke ist möglich. Durch mehrfache Anwendung des Verfahrens können alternativ oder zusätzlich zeitliche Entwicklungen des Feuchtigkeitswertes des Bodenareals und/oder der Feuchtigkeitswerte mehrerer Bodenareale dargestellt werden, zum Beispiel als zeitliches Verlaufsdiagramm.

Auch Werte der elektrischen physikalischen Größe untersuchter Bodenareale lassen sich auf einer Karte darstellen (insbesondere: elektrische Leitfähigkeit, Impedanz oder dielektrische Eigenschaften, insbesondere relative Dielektrizitätskonstante bzw. Permittivität). Auch jeweilige Laufzeiten des elektromagnetischen Signals können auf einer Karte oder als zweidimensionales Bild dargestellt werden. Auch eine Darstellung als Punktwolke ist möglich.

Das elektromagnetische Signal weist eine Frequenz auf, die
- in dem Frequenzbereich der Mittelwellen oder
- in dem Frequenzbereich der Langwellen oder
- nahe unterhalb des Frequenzbereichs der Langwellen oder
- in dem Frequenzbereich der Kurzwellen oder
- nahe oberhalb des Frequenzbereichs der Kurzwellen liegt.

Hierbei kann es sich um einen Frequenzanteil des elektromagnetischen Signals handeln. Es kann sich zum Beispiel um eine Frequenz eines Trägersignals handeln. Das elektromagnetische Signal kann mehrere Frequenzen aufweisen, die sich in den genannten Frequenzbereichen oder nahe unterhalb des Frequenzbereichs der Langwellen oder nahe oberhalb des Frequenzbereichs der Kurzwellen befinden.

Der Frequenzbereich der Langwellen umfasst per Definition Frequenzen zwischen 30 kHz und 300 kHz. Der Frequenzbereich der Mittelwellen umfasst per Definition Frequenzen zwischen 300 kHz bis 3000 kHz (3 MHz). Der Frequenzbereich der Kurzwellen umfasst per Definition Frequenzen zwischen 3 bis 30 MHz. Somit ergibt sich ein Gesamtfrequenzbereich von 30 kHz bis 30 MHz, der den Frequenzbereichen der Langwellen, der Mittelwellen oder der Kurzwellen zusammenfassend entspricht.

"Nahe" unterhalb des Frequenzbereichs der Langwellen kann zum Beispiel bedeuten, dass die untere Grenze des Frequenzbereichs der Langwellen um bis zu 2 kHz oder bis zu 5 kHz oder bis zu 10 kHz unterschritten werden kann. Die Untergrenze für die Frequenz kann dann bei 28 kHz, 25 kHz oder 20 kHz liegen. "Nahe" kann ebenfalls bedeuten, dass die untere Grenze des Frequenzbereichs der Langwellen insoweit unterschritten werden kann, als dass die Durchführung des erfindungsgemäßen Verfahrens noch sinnvoll möglich ist. Zu lange Wellen können die Exaktheit der Ermittlung des Wertes der physikalischen Eigenschaft des elektromagnetischen Signals negativ beeinträchtigen.

"Nahe" oberhalb des Frequenzbereichs der Kurzwellen kann zum Beispiel bedeuten, dass die obere Grenze des Frequenzbereichs der Kurzwellen um bis zu 2 MHz oder bis zu 5 MHz oder bis zu 10 MHz überschritten werden kann. Die Obergrenze für die Frequenz kann dann bei 32 MHz, 35 MHz oder 40 MHz liegen. "Nahe" kann ebenfalls bedeuten, dass die obere Grenze des Frequenzbereichs der Kurzwellen insoweit überschritten werden kann, als dass die Durchführung des erfindungsgemäßen Verfahrens noch sinnvoll möglich ist. Zu kurze Wellen werden als Bodenwellen stark gedämpft und können daher nur für Bodenareale eingesetzt werden, die eine vergleichsweise kurze Längsausdehnung aufweisen. Verwiesen wird an dieser Stelle nochmals auf Angulo et al., 2014, "Handbook on Ground Wave Propagation", ITU (International Telecommunication Union), Seite 9, Fig. 3.

Die Sendeeinrichtung kann eine Anlage zur Positionsbestimmung und zur Zeitbestimmung aufweisen, zum Beispiel einen Empfänger eines globalen Navigationssatellitensystems.

Die Empfangseinrichtung kann eine Anlage zur Positionsbestimmung und zur Zeitbestimmung aufweisen, zum Beispiel einen Empfänger eines globalen Navigationssatellitensystems. Hierüber ist ein genauer zeitlicher Abgleich, zum Beispiel eines Sendezeitpunkts und eines Empfangszeitpunkts möglich. Ein solcher zeitlicher Abgleich kann zur genauen Ermittlung des Wertes der physikalischen Eigenschaft genutzt werden, insbesondere, wenn es sich um einen Phasenwert oder einen Wert einer Laufzeit des elektromagnetischen Signals handelt.

Das vorgestellte Verfahren erlaubt eine Ermittlung der Bodenfeuchtigkeit von Bodenarealen verschiedener Größe (bis hin zu sehr großen Bodenarealen, die zum Beispiel eine Längsausdehnung von mehr als 100 km haben) zeitlich kontinuierlich, zuverlässig, flächendeckend und auf einfache und preisgünstige Art und Weise. Insbesondere können vorhandene Infrastrukturen, zum Beispiel vorhandene Sender, die Sendelizenzen aufweisen, genutzt werden. Insbesondere Empfangseinrichtungen können einfach und kostengünstig entweder an Land und/oder auf bestehender Infrastruktur zu Wasser (z. B. Bojen) oder Wasserfahrzeugen installiert werden. Weiterhin erlaubt das vorgestellte Verfahren eine einfache Skalierbarkeit, zum Beispiel durch Verwendung mehrerer Sendeeinrichtungen (z. B. mit jeweiligen Identifikationsmerkmalen im jeweiligen elektromagnetischen Signal) und/oder durch Verwendung mehrerer Empfangseinrichtungen zum Beispiel an unterschiedlichen zweiten Positionen. So kann auf einfache Art und Weise sowohl ein einziges Bodenareal als auch eine Mehrzahl an Bodenarealen, die ein zusammenhängendes Gebiet bilden können, in Bezug auf die Ermittlung von Feuchtigkeitswerten untersucht werden.

Das vorgestellte Verfahren unterscheidet sich von anderen bekannten Technologien insbesondere auch dadurch, dass es eine weitgehend topografieunabhängige Ermittlung von Feuchtigkeitswerten von Bodenarealen ermöglicht. Dieser Vorteil liegt insbesondere darin begründet, dass Bodenwellen sich grundsätzlich entlang und an Bodenoberflächen verbreiten.

Das vorgestellte Verfahren kann auch auf anderen Planeten oder auf Monden oder auf anderen Himmelskörpern (z. B. Asteroiden oder Planetoiden) angewendet werden.

In einer vorteilhaften Ausgestaltung des erfindungsgemäßen Verfahrens wird bei dem Ermitteln des Feuchtigkeitswerts des Bodenareals zusätzlich ein Referenzwert genutzt.

Varianten diese Ausgestaltung wurden bereits oben vorgestellt. Die Nutzung des Referenzwerts stellt eine besonders einfache und mit geringem programmiertechnischem

Aufwand und geringem Rechenaufwand umsetzbare Methode dar, die Genauigkeit des Feuchtigkeitswerts insbesondere mithilfe von Daten aus vorherigen Anwendungen des vorgestellten Verfahrens zu steigern.

In einer vorteilhaften Ausgestaltung des erfindungsgemäßen Verfahrens ist die physikalische Eigenschaft des elektromagnetischen Signals ein Phasenwert des elektromagnetischen Signals oder ein Wert einer Laufzeit des elektromagnetischen Signals.

Eine Veränderung des Phasenwertes - insbesondere kann der Phasenwert ein Phasenwert zu einem definierten Zeitpunkt sein - kann auf eine Veränderung der Ausbreitungsgeschwindigkeit und/oder der Wellenlänge des elektromagnetischen Signals hindeuten. Dies kann in der Folge eine Bestimmung des Feuchtigkeitswertes ermöglichen. Zur Bestimmung des Phasenwertes zu einem definierten Zeitpunkt sind besonders exakte Zeitmessungen erforderlich, die zum Beispiel durch Empfänger eines globalen Navigationssatellitensystems, die in Verbindung mit der Empfangseinrichtung und mit der Sendeeinrichtung stehen, realisiert werden können. Eine exakte Zeitsynchronisierung zwischen der Empfangseinrichtung und der Sendeeinrichtung kann notwendig sein. Der definierte Zeitpunkt kann zum Beispiel den Ablauf einer vordefinierten Zeitdauer nach dem Senden des elektromagnetischen Signals kennzeichnen. Der definierte Zeitpunkt kann ein zum Zeitpunkt des Sendens des elektromagnetischen Signals relativer Zeitpunkt sein.

Die Nutzung des Wertes der Laufzeit bietet den zusätzlichen Vorteil, dass ein Überspringen einer vollen Schwingung, das bei Nutzung des Phasenwertes nicht detektiert werden könnte, detektiert werden kann. Die Laufzeit kann zum Beispiel anhand eines spezifischen Merkmals des elektromagnetischen Signals gemessen werden, zum Beispiel eines charakteristischen Schwingungsmusters, das in Zeitabständen wiederholt wird.

Ein Phasenwert oder ein Wert der Laufzeit des elektromagnetischen Signals kann besonders einfach ermittelt oder gemessen und besonders einfach verarbeitet werden. Es ist auf einfache Art und Weise möglich, Phasenwerte oder Werte der Laufzeit bei verschiedenen Frequenzen des elektromagnetischen Signals zu messen, insbesondere, um den Feuchtigkeitswert durch Anwendung des erfindungsgemäßen Verfahrens bei verschiedenen Frequenzen des elektromagnetischen Signals besonders genau und/oder zuverlässig zu ermitteln.

In einer vorteilhaften Ausgestaltung des erfindungsgemäßen Verfahrens wird mithilfe des Wertes der physikalischen Eigenschaft des elektromagnetischen Signals ein Wert einer elektrischen physikalischen Größe des Bodenareals ermittelt und wird der Feuchtigkeitswert des Bodenareals mithilfe des Wertes der elektrischen physikalischen Größe des Bodenareals ermittelt.

Bemerkungen zu dieser Ausgestaltung wurden bereits oben vorgenommen. Die elektrische physikalische Größe kann insbesondere die elektrische Leitfähigkeit, die Impedanz oder eine dielektrische Eigenschaft, insbesondere die relative Dielektrizitätskonstante oder die Permittivität sein.

Zusammenhänge zwischen elektrischen physikalischen Eigenschaften von spezifischen Bodenarten und Feuchtigkeitswerten dieser Bodenarten sind in der Literatur bekannt. Die Berechnung von Feuchtigkeitswerten ist daher einfach und auch zuverlässig möglich. Die genannten Zusammenhänge ermöglichen insbesondere eine gute Ortsauflösung innerhalb des Bodenareals oder innerhalb eines Gebiets, das mehrere Bodenareale aufweist, auf die das erfindungsgemäße Verfahren angewendet wird, da unterschiedliche Bodenarten separat betrachtet werden können.

In einer vorteilhaften Ausgestaltung des erfindungsgemäßen Verfahrens ist die elektrische physikalische Größe des Bodenareals eine elektrische Leitfähigkeit oder eine Impedanz oder eine dielektrische Eigenschaft des Bodenareals. Die dielektrische Eigenschaft kann insbesondere die relative Dielektrizitätskonstante oder die Permittivität sein.

Die vormals genannten Vorteile gelten entsprechend. Vorteilhaft an der Nutzung der genannten elektrischen physikalischen Größen ist zudem, dass Zusammenhänge zwischen ihnen und Feuchtigkeitswerten von Bodenarten in der Literatur besonders gängig und für eine Vielzahl verschiedener Bodenarten bekannt sind. So können Feuchtigkeitswerte für vielfältige Bodenarten besonders einfach und zuverlässig ermittelt werden. Zudem sind Werte der genannten elektrischen physikalischen Größen mindestens teilweise durch andere Messverfahren ermittelbar. Dies erlaubt Möglichkeiten der Validierung des Wertes der elektrischen physikalischen Größe.

In einer vorteilhaften Ausgestaltung des erfindungsgemäßen Verfahrens wird der Wert der elektrischen physikalischen Größe des Bodenareals zusätzlich mithilfe von Bodendaten und/oder Bodenkarten des Bodenareals ermittelt.

Bodendaten und/oder Bodenkarten können insbesondere dazu dienen, unterschiedliche Bodenarten zu erkennen. So kann die Ortsauflösung innerhalb des Bodenareals oder innerhalb eines Gebiets, das mehrere Bodenareale aufweist, weiter verbessert werden. Zum Beispiel kann mithilfe des ermittelten Wertes der physikalischen Eigenschaft des elektromagnetischen Signals lediglich ein Durchschnittswert der elektrischen physikalischen Größe für ein betrachtetes Bodenareal ermittelbar sein.

Mithilfe von Bodendaten und/oder Bodenkarten kann dann jedoch anhand dieses Durchschnittswertes unter Betrachtung verschiedener Bodenarten in dem Bodenareal eine Differenzierung vorgenommen werden. So können Werte der elektrischen physikalischen Größe bezüglich verschiedener Bodenarten oder verschiedener Abschnitte des Bodenareals, die zu dem Durchschnittswert zwar beitragen, sich jedoch hinsichtlich ihres Wertes der elektrischen physikalischen Größe von dem Durchschnittswert selbst unterscheiden, bestimmt werden.

Auch ein Referenzwert, sofern er sich auf die elektrische physikalische Größe des Bodenareals bezieht, kann mithilfe von Bodendaten oder Bodenarten besonders zuverlässig ermittelt werden.

In einer vorteilhaften Ausgestaltung des erfindungsgemäßen Verfahrens wird der Wert der elektrischen physikalischen Größe des Bodenareals zusätzlich mithilfe eines Werts einer Temperatur des Bodenareals ermittelt.

Die Hinzuziehung des Wertes der Temperatur des Bodenareals ermöglicht eine Erhöhung der Genauigkeit beim Ermitteln des Wertes der elektrischen physikalischen Größe des Bodenareals. Hierdurch kann gleichsam die Genauigkeit der Ermittlung des Feuchtigkeitswerts gesteigert werden, insbesondere im jahreszeitlichen Kontext oder im Kontext einer Vegetationsperiode.

Auch die temperaturabhängige Ermittlung des Streuungsverhaltens oder des Absorptionsverhaltens innerhalb des Bodenareals ist zusätzlich im Rahmen des erfindungsgemäßen Verfahrens möglich und kann zum Beispiel bei der Ermittlung des Wertes der elektrischen physikalischen Größe und/oder eines Referenzwerts und/oder des Feuchtigkeitswertes genutzt werden. Eine temperaturabhängige Ermittlung des Streuungsverhaltens oder des Absorptionsverhaltens könnte mithilfe zahlreicher Anwendungen des erfindungsgemäßen Verfahrens zu unterschiedlichen Zeitpunkten erstellt werden.

In einer vorteilhaften Ausgestaltung des erfindungsgemäßen Verfahrens weist das elektromagnetische Signal ein Identifikationsmerkmal auf, das eine Identifikation der Sendeeinrichtung ermöglicht.

Das Identifikationsmerkmal kann eine vorgegebene Abfolge von elektromagnetischen Signalanteilen sein, die mithilfe der Empfangseinrichtung und/oder einer angeschlossenen oder integrierten Recheneinheit als Information erkennbar und/oder auslesbar ist. Das Identifikationsmerkmal kann zum Beispiel als Signalanteil auf einer Trägerfrequenz aufmoduliert sein. Es kann sich zum Beispiel um eine Ziffernfolge, zum Beispiel eine Nummer der Empfangseinrichtung, handeln. Es kann sich alternativ oder zusätzlich um Positionsdaten der Empfangseinrichtung handeln.

Eine Identifikation der Sendeeinrichtung ermöglicht es, dass eine einzige Empfangseinrichtung elektromagnetische Signale verschiedener Sendeeinrichtungen empfangen kann und eine Differenzierung der elektromagnetischen Signale möglich ist. Das heißt, dass mithilfe einer einzigen Empfangseinrichtung das erfindungsgemäße Verfahren für verschiedene Bodenareale durchgeführt werden kann. Dies stellt eine einfache Möglichkeit dar, große Gebiete, die mehrere Bodenareale beinhalten, mithilfe des erfindungsgemäßen Verfahrens in Bezug auf Feuchtigkeitswerte zu untersuchen. Die Empfangseinrichtung kann auch insoweit mehrere elektromagnetische Signale verschiedener Sendeeinrichtungen empfangen, als dass sie bewegbar oder in ihrer Position veränderbar sein kann, zum Beispiel auf einem Land- oder Wasserfahrzeug montiert sein kann. Hierbei ist die Differenzierung der elektromagnetischen Signale erforderlich.

Ein Datenkanal des elektromagnetischen Signals kann neben der Identifikation der Sendeeinrichtung und Positionsdaten auch weitere Daten enthalten wie z. B.: Fehlerkorrekturen (die zum Beispiel auf den Wert der physikalischen Eigenschaft des elektromagnetischen Signals anzuwenden sind), und oder Referenzwerte, zum Beispiel für ein bestimmtes Bodenareal. Weiterhin können zum Beispiel aktuelle Wetterdaten wie Temperaturwerte und/oder aktuell zu nutzende Modellparameter für die Ermittlung des Feuchtigkeitswerts effizient zur Verfügung gestellt werden.

Der Datenkanal kann auch zur Kommandierung der Empfangseinheit oder mehrerer Empfangseinheiten eingesetzt werden.

Der Datenkanal kann auch genutzt werden, um Informationen zwischen mehreren Sendeeinrichtungen auszutauschen, wenn mindestens eine Sendeeinrichtung auch über eine Empfangseinheit (z. B. eine Empfangseinrichtung) verfügt. Allgemein und nicht nur auf diese Ausgestaltung beschränkt können eine Empfangseinrichtung und eine Sendeeinrichtung zu einer gemeinsamen Einrichtung gekoppelt sein.

Bei dieser Ausgestaltung können neben präzisen Zweiwege-Messungen für das Bodenareal zwischen den Sendeeinrichtungen auch statische oder dynamische Sendeeinrichtungen mit Informationen für den Broadcast versorgt werden oder mithilfe von in-band-Synchronisation zeitlich mit einer Masterstation synchronisiert werden. Der Datenkanal kann auch Informationen über einen aktuellen Zustand der Sendeeinrichtung enthalten, die zum Beispiel für ein Kontrollzentrum oder einen Nutzer an der zweiten Position bestimmt sein können.

In einer vorteilhaften Ausgestaltung des erfindungsgemäßen Verfahrens ist/sind die Empfangseinrichtung und/oder die Sendeeinrichtung in ihrem Standort/ihren Standorten veränderbar.

Das heißt, die Empfangseinrichtung und/oder die Sendeeinrichtung ist konstruktiv derart ausgestaltet, dass sie ohne großen Aufwand an einen anderen Standort verbracht werden kann. Die Empfangseinrichtung und/oder die Sendeeinrichtung kann/können zum Beispiel transportabel sein. Zum Beispiel kann sie auf einem transportablen Träger und/oder einem Land- und/oder einem Wasserfahrzeug und/oder einem schwimmfähigen Gegenstand montierbar sein. Insbesondere kann eine Antenne der Empfangseinrichtung und/oder der Sendeeinrichtung derart ausgestaltet sein, dass sie nicht mit dem Boden verbunden wird und/oder dass sie eine lösbare Verbindung mit dem Boden eingeht.

Die Ausgestaltung ist vorteilhaft, weil das erfindungsgemäße Verfahren für verschiedene Bodenareale mit derselben Empfangseinrichtung und/oder derselben Sendeeinrichtung durchgeführt werden kann. Dies bewirkt insbesondere eine Aufwands- und Kostenersparnis.

Das elektromagnetische Signal kann (allgemein, nicht nur auf die vorgestellte Ausgestaltung begrenzt) Daten und/oder Informationen enthalten, zum Beispiel Informationen über eine aktuelle Position der Sendeeinrichtung oder der Empfangseinrichtung, eine Identifikation der Sendeeinrichtung und/oder Statusinformationen und/oder anzuwendende Fehlerkorrekturen. Nach einer Ortsveränderung der Sendeeinrichtung oder der Empfangseinrichtung können solche Informationen neu zu bestimmen sein.

In einer vorteilhaften Ausgestaltung des erfindungsgemäßen Verfahrens ist/sind die Empfangseinrichtung und/oder die Sendeeinrichtung jeweils auf einem Landfahrzeug oder einem Wasserfahrzeug angeordnet.

Diese Ausgestaltung ermöglicht ebenfalls, dass die Empfangseinrichtung und/oder die Sendeeinrichtung ohne großen Aufwand an einen anderen Standort verbracht werden kann.

Die Ausgestaltung ist vorteilhaft, weil das erfindungsgemäße Verfahren für verschiedene Bodenareale mit derselben Empfangseinrichtung und/oder derselben Sendeeinrichtung durchgeführt werden kann. Dies bewirkt insbesondere eine Aufwands- und Kostenersparnis. Zudem kann eine besondere Aufwands- und Kostenersparnis erzielt werden, wenn die Empfangseinrichtung und/oder die Sendeeinrichtung auf einem sich ohnehin bewegenden Landfahrzeug oder Wasserfahrzeug angeordnet wird. Es kann sich zum Beispiel um ein Schiff, zum Beispiel eine Fähre, im Linienverkehr handeln. Hierdurch kann einerseits auf einfache und kostengünstige Art und Weise ein großes Gebiet mit einer Vielzahl von Bodenarealen untersucht werden. Andererseits können auf einfache und kostengünstige Art und Weise zeitlich wiederkehrende Anwendungen des erfindungsgemäßen Verfahrens durchgeführt werden. Hierdurch kann eine kontinuierliche zeitliche Entwicklung des Feuchtigkeitswerts erreicht werden.

In einer vorteilhaften Ausgestaltung des erfindungsgemäßen Verfahrens wird/werden ein Wert einer Energiedichte des elektromagnetischen Signals und/oder ein Wert einer Leistung des elektromagnetischen Signals ermittelt und wird der Feuchtigkeitswert des Bodenareals zusätzlich mithilfe des Wertes der Energiedichte und/oder des Wertes der Leistung ermittelt.

Die Ermittlung des Wertes der Energiedichte und/oder der Leistung ist, zum Beispiel mithilfe der Empfangseinrichtung, technisch leicht möglich. Mithilfe dieses Wertes/dieser Werte kann zusätzlich insbesondere eine Berechnung eines Wertes einer dielektrischen Eigenschaft (insbesondere Permittivität oder relative Dielektrizitätskonstante) oder einer Impedanz oder eines Werts, der ein Streuungsverhalten oder ein Absorptionsverhalten beschreibt, vorgenommen werden. Mithilfe mindestens eines der genannten Werte kann die Ermittlung des Feuchtigkeitswerts von der Genauigkeit her verbessert werden.

In einer vorteilhaften Ausgestaltung des erfindungsgemäßen Verfahrens ist eine Mehrzahl von Sendeeinrichtungen an verschiedenen Standorten und/oder eine Mehrzahl von Empfangseinrichtungen an verschiedenen Standorten vorgesehen und wird das Verfahren mithilfe der Mehrzahl von Sendeeinrichtungen und/oder der Mehrzahl von Empfangseinrichtungen für eine Mehrzahl von Bodenarealen durchgeführt.

Zum Beispiel kann eine Mehrzahl von Empfangseinrichtungen und eine Sendeeinrichtung entlang eines linienartigen Pfades auf dem Boden angeordnet sein und kann das erfindungsgemäße Verfahren für eine Mehrzahl von Bodenarealen zeitgleich oder nacheinander durchgeführt werden, die Abschnitte der Linie umfassen. Die Linie kann Höhenunterschiede - zum Beispiel Berge und Täler - auf dem Boden aufweisen, sich aber in der Horizontalen linienartig erstrecken.

Zum Beispiel kann eine Mehrzahl von Empfangseinrichtungen um eine Sendeeinrichtung herum angeordnet sein und das Verfahren für eine Mehrzahl von Bodenarealen durchgeführt werden, die sich jeweils zwischen der Sendeeinrichtung und den Empfangseinrichtungen befinden.

Zum Beispiel können mehrere Sendeeinrichtungen und mehrere Empfangseinrichtungen (zum Beispiel gleichmäßig) entlang gegenüberliegenden Seiten eines zu untersuchenden großflächigen Bodengebiets, das mehrere separat zu untersuchende Bodenareale aufweist, angeordnet sein. Die geraden Bodenpfade (jeweiligen Abschnitte eines Großkreises) zwischen einer Sendeeinrichtung und einer Empfangseinrichtung schneiden mehrfach die zu untersuchenden Bodenareale. Hierdurch kann die Genauigkeit von ermittelten Feuchtigkeitswerten erhöht werden. Die Anzahl an Sendeeinrichtungen und Empfangseinrichtungen hat Einfluss auf eine zweidimensionale Ortsauflösung von Bodenfeuchtewerten. Empfangseinrichtungen können auch in ihren Standorten veränderbar sein oder verändert werden.

Die vorgestellte Ausgestaltung ist vorteilhaft, weil besonders große Gebiete (aufweisend eine Mehrzahl von zu untersuchenden Bodenarealen, die zusammenhängen können) mit hoher Ortsauflösung mithilfe des erfindungsgemäßen Verfahrens untersucht werden können. Sendeeinrichtungen oder Empfangseinrichtungen können zum Beispiel an Grenzbereichen zwischen verschiedenen Bodenarten angeordnet sein. So können Bodenareale, die jeweils eine einzige Bodenart oder jeweils nur wenige Bodenarten aufweisen, separat untersucht werden. Es ist nicht ausgeschlossen, dass der Standort von Sendeeinrichtungen oder Empfangseinrichtungen veränderlich ist, wodurch die Größe des Gebiets oder die Ortsauflösung weiter gesteigert werden kann bei gleichbleibender Anzahl von Sendern und Empfängern.

Die vorgestellte Ausgestaltung erlaubt es zudem, Laufzeitkarten oder -diagramme in Bezug auf die Laufzeit des elektromagnetischen Signals in einem Gebiet zu erstellen.

In einer vorteilhaften Ausgestaltung des erfindungsgemäßen Verfahrens wird
- die Frequenz des elektromagnetischen Signals zu einer weiteren Frequenz verändert und
- wird mindestens ein weiterer Wert der physikalischen Eigenschaft des elektromagnetischen Signals bei der weiteren Frequenz ermittelt und wird
- mithilfe des Weiteren Werts der physikalischen Eigenschaft ein weiterer Feuchtigkeitswert des Bodenareals ermittelt.

Die weitere Frequenz kann eine Frequenz sein, die
- in dem Frequenzbereich der Mittelwellen oder
- in dem Frequenzbereich der Langwellen oder
- nahe unterhalb des Frequenzbereichs der Langwellen oder
- in dem Frequenzbereich der Kurzwellen oder
- nahe oberhalb des Frequenzbereichs der Kurzwellen liegt.

Die Sendeeinrichtung kann ausgestaltet sein, elektromagnetische Signale mit unterschiedlichen Frequenzen zu senden. Mehrere Feuchtigkeitswerte des Bodenareals bei unterschiedlichen Frequenzen können ermittelt werden, sodass, unter Berücksichtigung der Feuchtigkeitswerte verbesserte Aussagen über das Feuchtigkeitsverhalten des Bodenareals getroffen werden können. Insbesondere ermöglicht die Ausgestaltung das Bodenareal in verschiedenen Schichttiefen zu analysieren. Auch lässt sich so, zum Beispiel durch Mittelwertbildung mehrerer Feuchtigkeitswerte, ein genauerer Feuchtigkeitswert, der auf einer vergrößerten Informationsbasis beruht, ermitteln.

Die Ausgestaltung ist weiterhin vorteilhaft, weil sich dadurch mehr Bodeninformationen pro Sendeeinrichtung ableiten lassen. Dies kann auch dazu genutzt werden, die Dichte von Sendeeinrichtungen zu reduzieren.

Der Ausgestaltung lässt sich noch erweitern, sodass eine Empfangseinrichtung oder eine zentrale Kontrolleinheit gezielt die Sendeeinrichtung steuert oder sie veranlasst, ein elektromagnetisches Signal in einer bestimmten Frequenz zu senden, um optimal das Bodenareal hinsichtlich Genauigkeit und Schichtdicke analysieren zu können.

Empfangseinrichtungen, die sich näher an einer Sendeeinrichtung befinden, können mit hochfrequenteren elektrischen Signalen arbeiten, da bei einer geringen Entfernung zwischen der Sendeeinrichtung und der Empfangseinrichtung die Dämpfung vergleichsweise gering ist. Die Genauigkeit des Feuchtigkeitswert ist bei hochfrequenteren elektrischen Signalen erhöht.

Empfangseinrichtungen, die sich weiter von einer Sendeeinrichtung entfernt befinden, können mit niederfrequenteren elektrischen Signalen arbeiten, da bei einer höheren Entfernung zwischen der Sendeeinrichtung und der Empfangseinrichtung die Dämpfung dann geringer ist als bei hochfrequenten elektrischen Signalen.

Gemäß Anspruch 13 wird ein System zur Durchführung des erfindungsgemäßen Verfahrens zur Ermittlung von Feuchtigkeitswerten eines Bodenareals vorgeschlagen, das die folgenden und weitere Merkmale aufweist:
- eine Sendeeinrichtung, die ausgestaltet ist, ein elektromagnetisches Signal derart zu senden, dass es sich als Bodenwelle verbreitet, und die ausgestaltet ist, das elektromagnetische Signal mit einer Frequenz zu senden, die in dem Frequenzbereich der Mittelwellen oder in dem Frequenzbereich der Langwellen oder nahe unterhalb des Frequenzbereichs der Langwellen oder in dem Frequenzbereich der Kurzwellen oder nahe oberhalb des Frequenzbereichs der Kurzwellen liegt.
- eine Empfangseinrichtung, die ausgestaltet ist, das elektromagnetische Signal zu empfangen;
- eine Ermittlungseinrichtung, die ausgestaltet ist, je einen Wert von zumindest zwei physikalischen Eigenschaften des elektromagnetischen Signals zu ermitteln;
wobei die Ermittlungseinrichtung zusätzlich ausgestaltet ist, Feuchtigkeitswerte einer Mehrzahl von Teilbereichen des Bodenareals basierend auf dem Modell zu ermitteln.

In Bezug auf mögliche Ausgestaltungen des Systems wird vollinhaltlich auf die Ausführungen zum erfindungsgemäßen Verfahren Bezug genommen und umgekehrt.

Die Sendeeinrichtung kann insbesondere einen Sender (einen Transmitter) aufweisen, also eine Einrichtung zur Erzeugung und Abstrahlung von elektromagnetischen Wellen. Die Sendeeinrichtung kann insbesondere einen Oszillator, einen Verstärker, eine Energieversorgungseinrichtung und/oder eine Sendeantenne aufweisen. Sie kann eine Recheneinheit aufweisen oder mit einer Recheneinheit (zum Beispiel einen Computer) verbindbar sein. Die Sendeeinrichtung kann mit dem Boden elektrisch verbindbar sein. Insbesondere kann die Sendeeinrichtung eine Antenne aufweisen, die mit dem Boden elektrisch verbindbar sein kann. Die Sendeeinrichtung kann eine Positionsbestimmungsvorrichtung und/oder eine Zeitbestimmungsvorrichtung aufweisen, zum Beispiel einen Empfänger eines globalen Navigationssatellitensystems oder eine Uhr oder einen Empfänger eines sonstigen (z. B. terrestrischen) Navigationssystems oder einen Empfänger einer Uhrzeit, oder mit einer solchen Vorrichtung verbunden sein. Diese Vorrichtung kann ausgestaltet sein, einen exakten Zeitpunkt zu ermitteln, zu dem das elektromagnetische Signal abgesendet wird. Eine Positionsbestimmungsvorrichtung und/oder eine Zeitbestimmungsvorrichtung erlaubt besonders exakte Ermittlungen des Feuchtigkeitswerts auf einfache Art und Weise.

Die Empfangseinrichtung kann insbesondere einen Empfänger (einen Receiver) aufweisen. Insbesondere kann der Empfänger eine Antenne, einen Filter, einen Verstärker und/oder eine Energieversorgungseinrichtung aufweisen. Die Empfangseinrichtung kann eine Recheneinheit aufweisen oder mit einer Recheneinheit (zum Beispiel einem Computer) verbindbar sein. Die Empfangseinrichtung kann mit dem Boden elektrisch verbindbar sein. Insbesondere kann die Empfangseinrichtung eine Antenne aufweisen, die mit dem Boden elektrisch verbindbar sein kann. Die Empfangseinrichtung kann eine Positionsbestimmungsvorrichtung und/oder eine Zeitbestimmungsvorrichtung aufweisen, zum Beispiel einen Empfänger eines globalen Navigationssatellitensystems oder eine Uhr oder einen Empfänger eines sonstigen (z. B. terrestrischen) Navigationssystems oder einen Empfänger einer Uhrzeit, oder mit einer solchen Vorrichtung verbunden sein. Diese Vorrichtung kann ausgestaltet sein, einen exakten Zeitpunkt zu ermitteln, zu dem das elektromagnetische Signal empfangen wird. Alternativ oder zusätzlich kann diese Vorrichtung ausgestaltet sein, den Wert der physikalischen Größe (zum Beispiel einen Phasenwinkel) zu einem exakten Zeitpunkt zu ermitteln, zum Beispiel zu jeder vollen Sekunde. Eine Positionsbestimmungsvorrichtung und/oder eine Zeitbestimmungsvorrichtung erlaubt besonders exakte Ermittlungen des Feuchtigkeitswerts auf einfache Art und Weise.

Die Ermittlungseinrichtung kann insbesondere mindestens eine Recheneinheit, insbesondere zum Beispiel mindestens einen Computer, aufweisen. Die Ermittlungseinrichtung kann mehrere Recheneinheiten aufweisen. Verschiedene Komponenten der Ermittlungseinrichtung können derart ausgestaltet sein, dass sie räumlich an oder bei der Empfangseinrichtung und/oder entfernt davon anzuordnen sein können. Zum Beispiel kann eine Recheneinheit an der Empfangseinrichtung angeordnet sein. Eine weitere Recheneinheit kann entfernt davon, zum Beispiel in einem anderen Gebäude oder an einem anderen Ort angeordnet sein. Zwischen der Recheneinheit und der weiteren Recheneinheit kann zum Beispiel eine drahtlose oder drahtgebundene datentechnische Verbindung bestehen. Es ist auch nicht ausgeschlossen, dass keine Daten technische Verbindung besteht und dass Daten zum Beispiel mithilfe eines Speichers zwischen der Recheneinheit und der weiteren Recheneinheit ausgetauscht werden.

Der mindestens ein Computer kann insbesondere eine CPU, einen Speicher, eine drahtlose und/oder eine drahtgebundene Datenschnittstelle (zum Beispiel zur Verbindung mit der Sendeeinrichtung und/oder mit der Empfangseinrichtung), ein Eingabegerät und/oder ein Ausgabegerät und eine Einrichtung zur Energieversorgung aufweisen.

Die Ermittlungseinrichtung kann insbesondere eine Auswerteeinrichtung aufweisen. Die Auswerteeinrichtung kann einen zeitlichen Synchronizer aufweisen, der ausgestaltet ist, die zeitliche Synchronizität des Sendens und des Empfangens des elektromagnetischen Signals herzustellen. Die Auswerteeinrichtung kann eine Routine zur Ermittlung des Phasenwertes oder des Wertes der Laufzeit aufweisen. Weiterhin kann sie Routinen aufweisen, die zur Umrechnung des Phasenwertes, des Amplitudenwertes und/oder des Wertes der Laufzeit hin zu dem Feuchtigkeitswert dienen, ggf. über den Zwischenschritt der Bestimmung eines Wertes der elektrischen physikalischen Größe des Bodenareals und/oder zum Beispiel unter Berücksichtigung eines Referenzwerts. Mithilfe eines vorgegebenen Bodenfeuchtemodells kann der Feuchtigkeitswertes des Bodenareals ermittelbar sein.

Alle zuvor getroffenen Aussagen über Maßangaben (z. B. Höhen, Längen und Breiten) verstehen sich als Beispiele für mögliche oder bevorzugte Ausgestaltungen des erfindungsgemäßen Verfahrens und des erfindungsgemäßen Systems. Sie stellen keine Einschränkungen, insbesondere im Hinblick auf Funktionalitäten, des erfindungsgemäßen Verfahrens oder des erfindungsgemäßen Systems dar. Eine von den angegebenen Maßangaben abweichende Auslegung des erfindungsgemäßen Verfahrens oder des erfindungsgemäßen Systems ist daher möglich. Die Aussagen sind nicht als einschränkend zu verstehen.

Ausführungsbeispiele der Erfindung werden nun unter Bezugnahme auf die beigefügte Zeichnung beschrieben. Die einzelnen Figuren der Zeichnung zeigen:
- Fig. 1A:: eine schematische Ansicht eines Ausführungsbeispiels des erfindungsgemäßen Verfahrens;
- Fig. 1B:: eine schematische Ansicht eines weiteren Ausführungsbeispiels des erfindungsgemäßen Verfahrens;
- Fig. 2A:: eine schematische Ansicht eines erfindungsgemäßen Systems und eines zu untersuchenden Bodenareals;
- Fig. 2B:: eine schematische Ansicht eines weiteren erfindungsgemäßen Systems und eines zu untersuchenden Bodenareals;
- Fig. 3:: eine schematische Ansicht eines senderseitigen Teilaspekts des in Fig. 2A gezeigten erfindungsgemäßen Systems;
- Fig. 4:: eine schematische Ansicht eines empfängerseitigen Teilaspekts des in Fig. 2A gezeigten erfindungsgemäßen Systems;
- Fig. 5A:: eine schematisch dargestellte mögliche Anordnung mehrerer Sendeeinrichtungen und mehrerer Empfangseinrichtungen;
- Fig. 5B:: eine schematisch dargestellte mögliche Anordnung mehrerer Sendeeinrichtungen und mehrerer Empfangseinrichtungen;
- Fig. 5C:: eine schematisch dargestellte mögliche Anordnung mehrerer Sendeeinrichtungen und mehrerer Empfangseinrichtungen.

In verschiedenen Ausführungsbeispielen werden gleiche Bezugszeichen für funktionsgleiche Einrichtungen, Schritte, Instanzen und Elemente verwendet. Dies bedeutet jedoch nicht zwangsläufig, dass es sich bei den Ausführungsbeispielen um dasselbe Ausführungsbeispiel oder Teile desselben Ausführungsbeispiels handelt.

Fig. 1A zeigt eine schematische Ansicht eines Ausführungsbeispiels des erfindungsgemäßen Verfahrens. In einem ersten Schritt S1 wird ein elektromagnetisches Signal SI1 mithilfe einer Sendeeinrichtung, die zum Beispiel die Sendeeinrichtung 1 (vgl. Fig. 2A, 3) sein kann, ausgesendet. Die Frequenz des elektromagnetischen Signals SI1 liegt zum Beispiel im Frequenzbereich der Kurzwellen, im Frequenzbereich der Mittelwellen oder im Frequenzbereich der Langwellen. Es ist auch möglich, dass die Frequenz des elektromagnetischen Signals SI1 nahe unterhalb des Frequenzbereichs der Langwellen liegt aber nicht in ihm oder nahe oberhalb des Frequenzbereichs der Kurzwellen liegt aber nicht in ihm. Konkret kann die Frequenz des elektromagnetischen Signals zum Beispiel bei 1 MHz liegen und damit im Frequenzbereich der Mittelwelle.

Die Sendeeinrichtung 1 ist ausgestaltet, das elektromagnetische Signal SI1 als Bodenwelle auszusenden. Weitere Ausführungen zur Sendeeinrichtung 1 folgen weiter unten. Es ist nicht ausgeschlossen und insbesondere bei vergleichsweise geringeren Wellenlängen bzw. höheren Frequenzen auch zu erwarten, dass ein gewisser Anteil der durch die Sendeeinrichtung 1 abgestrahlten elektrischen Energie sich auch in Form einer Raumwelle fortbewegt. Eine solche Raumwelle hat für das erfindungsgemäße Verfahren jedoch keine Relevanz; sie stellt eine Begleiterscheinung dar.

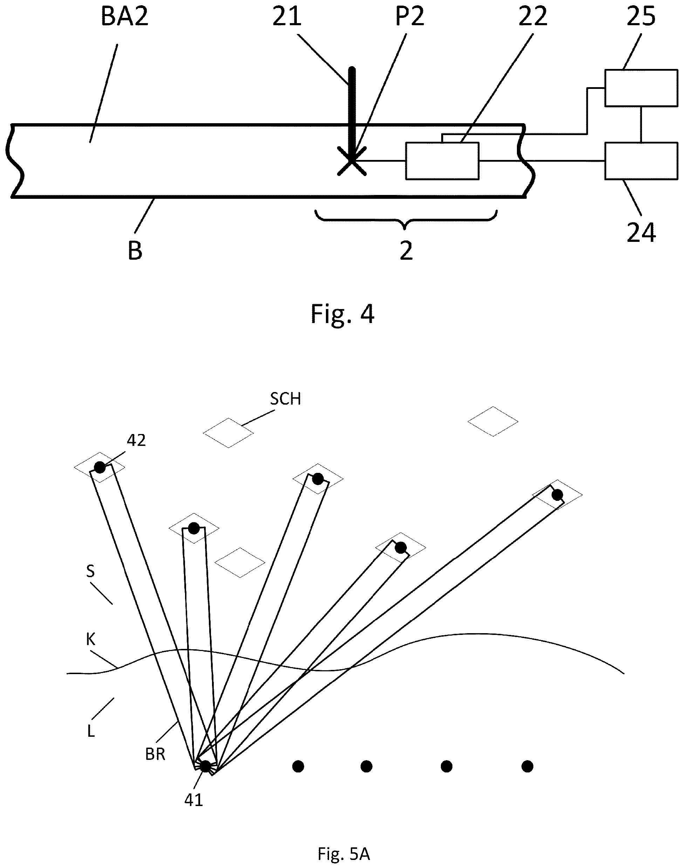

Das elektromagnetische Signal SI1 kann ein Identifikationsmerkmal aufweisen, insbesondere ein solches Identifikationsmerkmal, das einen Rückschluss auf die Sendeeinrichtung 1 erlaubt. Das Identifikationsmerkmal kann zum Beispiel ein in Form von auf ein Trägersignal aufmodulierten Wellen bestehender Signalanteil des Signals SI1 sein und inhaltlich zum Beispiel eine Nummer der Sendeeinrichtung 1 enthalten. Das elektromagnetische Signal SI1 kann auch Informationen über den geographischen Ort der ersten Position, die zum Beispiel die erste Position P1 (vgl. Fig. 2A, 3) sein kann, und/oder Informationen über einen Sendezeitpunkt oder über Fehlerkorrekturen oder über Referenzwerte aufweisen. Auch weitere Informationen sind möglich, zum Beispiel Wetterdaten, Temperaturwerte und/oder aktuell zu nutzende Modellparameter. Informationen über den Sendezeitpunkt oder den geographischen Ort der ersten Position können zum Beispiel mithilfe eines Empfängers eines Navigationssystems, zum Beispiel eines terrestrischen Navigationssystems oder globalen/regionalen Navigationssatellitensystems, zum Beispiel dem senderseitigen Empfänger 15 eines globalen Navigationssatellitensystems (vgl. Fig. 2A, 3), ermittelt werden. In Bezug auf den geographischen Ort der ersten Position kann alternativ auch eine geodätische Einmessung erfolgen. Die Informationen können mithilfe des elektromagnetischen Signals SI1 oder auf andere Art und Weise zum Zwecke einer Verarbeitung, insbesondere zur Ermittlung eines Phasenwertes PH1, an eine Ermittlungseinrichtung, zum Beispiel die Ermittlungseinrichtung 24 (vgl. Fig. 2A, 4), weitergeleitet werden. Der Phasenwert PH1 kann sich allgemein zum Beispiel auf eine elektrische oder eine magnetische Feldkomponente des elektromagnetischen Signals SI1 beziehen.

Die erste Position P1, an der sich eine Sendeantenne, zum Beispiel die Sendeantenne 11 (vgl. Fig. 2A, 3), befindet, kann eine Position an Land und direkt an der Bodenoberfläche des Bodens sein, zum Beispiel des Bodens B (vgl. Fig. 3). Es ist allgemein nicht ausgeschlossen, dass die erste Position P1 im Zuge mehrerer Anwendungen des erfindungsgemäßen Verfahrens veränderlich ist. Um dies zu erreichen kann die Sendeeinrichtung 1 mobil und/oder transportabel ausgestaltet sein. Zum Beispiel kann sie auf einem mobilen Träger angeordnet sein. Sie kann alternativ oder zusätzlich auf einem Landfahrzeug oder einem Seefahrzeug oder einer schwimmfähigen Tragvorrichtung montierbar oder montiert sein.

Die Sendeantenne 11, die ein Teil der Sendeeinrichtung 1 ist, kann sich teilweise in den Boden B erstrecken, wie in Fig. 3 dargestellt ist, und/oder mit dem Boden B elektrisch leitend verbunden sein. Die Sendeleistung kann so besonders effektiv zur Verbreitung als Bodenwelle genutzt werden. Die Sendeantenne 11 kann insbesondere zum Beispiel eine Monopol- oder eine Dipolantenne sein.

Bei einer Frequenz von 1 MHz ergibt sich eine Wellenlänge von 300 m. Diese Wellenlänge kann genutzt werden zur Anwendung des erfindungsgemäßen Verfahrens bei verschiedenen Größenordnungen eines zu untersuchenden Bodenareals, zum Beispiel des Bodenareals BA1 (vgl. Fig. 2A).

Eine sich zwischen der ersten Position P1 und einer zweiten Position, zum Beispiel der zweiten Position P2 (vgl. Fig. 2A, 4), erstreckende Längsausdehnung des Bodenareals BA1 kann zum Beispiel zwischen 10 km bis 200 km liegen (vgl. z. B. Angulo et al., 2014, "Handbook on Ground Wave Propagation", ITU (International Telecommunication Union), Seite 9, Fig. 3). Auch Längsausdehnungen darüber oder darunter sind nicht ausgeschlossen. An der genannten Stelle des "Handbook on Ground Wave Propagation" ist ein beispielhafter Abfall der Feldstärke über der Distanz für verschiedene Wellenlängen dargestellt. Derartige Zusammenhänge können bei der Wahl einer passenden Wellenlänge und/oder einer passenden Sendeleistung im Vorfeld der Anwendung des erfindungsgemäßen Verfahrens hilfreich sein.

Die Breite des Bodenareals BA1, auf die ein letztendlich ermittelter Feuchtigkeitswert F1 zumindest durchschnittlich zutrifft, braucht nicht genau bestimmt zu werden. Verwiesen wird auf die Ausführungen hierzu im allgemeinen Teil der Beschreibung. Insbesondere kann sich das Bodenareal BA1 eine Gesamtbreite von zwei Fresnel-Halbwellen-Zonen aufweisen. Die Breite ist geringer als die Längsausdehnung, sie könnte zum Beispiel zwischen 1 Meter bis 10 km betragen. Auch die Tiefe des Bodenareals BA1, auf die der letztendlich ermittelte Feuchtigkeitswert F1 zutrifft, braucht nicht genau bestimmt zu werden. Verwiesen wird auf die Ausführungen hierzu im allgemeinen Teil der Beschreibung. Die Tiefe könnte zum Beispiel 2 Meter bis 20 Meter betragen.

In einem zweiten Schritt S2 wird das elektromagnetische Signal SI1 empfangen mithilfe einer Empfangseinrichtung, die zum Beispiel die Empfangseinrichtung 2 (vgl. Fig. 2A, 4) sein kann. Die Empfangseinrichtung 2 ist ausgestaltet, das elektromagnetische Signal SI1 als Bodenwelle zu empfangen. Weitere Ausführungen zur Empfangseinrichtung 2 folgen weiter unten.

Die zweite Position P2, an der sich eine Empfangsantenne, zum Beispiel die Empfangsantenne 21 (vgl. Fig. 2A, 4), befindet, kann eine Position an Land und direkt an der Bodenoberfläche des Bodens sein, zum Beispiel des Bodens B (vgl. Fig. 4). Die Empfangsantenne 21 kann zum Beispiel eine elektrische Monopolantenne oder eine elektrische Dipolantenne und/oder eine magnetische Antenne und/oder eine Ferritantenne sein. Es kann sich um eine E-Feld-Antenne oder eine H-Feld-Antenne (z. B. eine Loop-Antenne) handeln. Die Empfangsantenne 21 kann auch oberhalb des Bodens B befindlich sein, zum Beispiel 0 bis 5 Meter, oder (zum Beispiel in einem Fall, wenn sich die Empfangsantenne auf einem großen Schiff befindet), mehr als 5 Meter, zum Beispiel 10 Meter, 20 Meter, 50 Meter oder 100 Meter. Die Empfangsantenne 21 (und ggf. auch die Empfangseinrichtung 2) kann zum Beispiel auf einem mobilen Träger oder an Bord eines Wasserfahrzeugs oder Seefahrzeugs befindlich sein.

Es ist allgemein nicht ausgeschlossen, dass die zweite Position P2 im Zuge mehrerer Anwendungen des erfindungsgemäßen Verfahrens veränderlich ist. Um dies zu erreichen kann die Empfangseinrichtung 2 mobil und/oder transportabel ausgestaltet sein. Zum Beispiel kann sie auf einem mobilen Träger angeordnet sein. Sie kann alternativ oder zusätzlich auf einem Landfahrzeug oder einem Seefahrzeug oder einer schwimmfähigen Tragvorrichtung montierbar oder montiert sein.

Die Empfangsantenne 21, die ein Teil der Empfangseinrichtung 2 ist, kann sich direkt oberhalb des Bodens senkrecht erstrecken, wie in Fig. 4 dargestellt ist. Alternativ kann sich die Empfangsantenne auch in den Boden B hineinerstrecken und/oder mit dem Boden B elektrisch leitend verbunden sein. Wenn die Empfangsantenne 21 eine H-Feld-Antenne ist, wird sie mit dem Boden nichtleitend verbunden.

Das Bodenareal BA1 kann von der Bodenart her homogen beschaffen sein, zum Beispiel aus einer Lehmschicht oder einer Sandschicht oder einer Waldbodenschicht oder einer Tonschicht bestehen oder im Wesentlichen aus einer der genannten Schichten bestehen. Das Bodenareal BA1 kann alternativ heterogen beschaffen sein, zum Beispiel einen Bereich aus Lehm und/oder einen Bereich aus Sand und/oder einen Bereich aus Waldboden und/oder einen Bereich aus Ton aufweisen.

Im zweiten Schritt S2 können zusätzlich insbesondere ein Empfangszeitpunkt des Signals SI1 und/oder Informationen über den geographischen Ort der zweiten Position P2 ermittelt werden. Dies kann zum Beispiel mithilfe eines Empfängers eines globalen Navigationssatellitensystems, zum Beispiel dem empfängerseitigen Empfänger 25 (vgl. Fig. 2A, 4) eines globalen Navigationssatellitensystems erfolgen.

In einem dritten Schritt S3 wird der Phasenwert PH1 des elektromagnetischen Signals SI1 ermittelt. Das elektromagnetische Signal SI1 kann mehrere Signalkomponenten aufweisen, zum Beispiel mehrere Sinussignale und/oder einen oder mehrere aufmodulierte Datenkanäle. Der Phasenwert PH1 kann von einer dieser Signalkomponenten bestimmt werden. Auch mehrere Phasenwerte, von mehreren dieser Signalkomponenten, die zur weiteren Verarbeitung und Ausführung des erfindungsgemäßen Verfahrens wie beschrieben genutzt werden können, können bestimmt werden.

Die Ermittlung des Phasenwerts PH1 (oder ggf. mehrerer Phasenwerte) kann mithilfe einer Ermittlungseinrichtung erfolgen, zum Beispiel der Ermittlungseinrichtung 24 (vgl. Fig. 2A, 4). Der Phasenwert PH1 kann insbesondere zu einem vordefinierten Zeitpunkt, zum Beispiel einem vordefinierten Zeitpunkt nach Absenden des elektromagnetischen Signals SI1, gemessen werden.

In einem vierten Schritt S4 wird mithilfe des Phasenwerts PH1 ein Wert EL1 der elektrischen Leitfähigkeit des Bodenareals BA1 ermittelt. Hierbei kann ein Referenzphasenwert RP1 und/oder können weitere Referenzphasenwerte hinzugezogen werden. Der Wert EL1 kann zum Beispiel durch Vergleich oder Abgleich mit dem Referenzphasenwert RP1 und ggf. weiteren Referenzphasenwerten ermittelt werden.

Der Referenzphasenwert RP1 kann zum Beispiel unter Berücksichtigung
- der Längsausdehnung des Bodenareals BA1 und/oder
- einer bekannten (zum Beispiel experimentell ermittelten) Signalausbreitungsgeschwindigkeit
   - in einer bestimmten Bodenart und/oder
   - bei einem bekanntem (zum Beispiel experimentell ermittelten) Wert der elektrischen Leitfähigkeit und/oder
   - bei einer Frequenz, die der Frequenz des elektromagnetischen Signals SI1 entspricht,
rechnerisch ermittelt worden sein, wobei die Bodenart der Bodenart des Bodenareals BA1 entsprechen kann.

Der Referenzphasenwert RP1 kann auch zum Beispiel unter Nutzung des vorgestellten Systems ermittelt, insbesondere gemessen worden sein, wobei zudem die Längsausdehnung, die Bodenart, der Wert der elektrischen Leitfähigkeit und/oder die Frequenz bekannt waren bzw. ebenfalls gemessen worden sind. Auch können mehrere Referenzphasenwerte bei unterschiedlichen Werten der elektrischen Leitfähigkeit für die zutreffende Bodenart bekannt sein. Mehrere Referenzphasenwerte bei unterschiedlichen Werten der elektrischen Leitfähigkeit können im vierten Schritt S4 zur Ermittlung des Wertes EL1 der elektrischen Leitfähigkeit genutzt werden, falls erforderlich oder gewünscht. Die Ermittlung des Wertes EL1 kann zum Beispiel mithilfe der Ermittlungseinrichtung 24 und entsprechender Programmroutinen erfolgen.

In einem fünften Schritt S5 wird anhand des Wertes EL1 der elektrischen Leitfähigkeit mittels bekannter Zusammenhänge der Wert F1 der Bodenfeuchtigkeit des Bodenareals BA1 ermittelt, zum Beispiel anhand von bekannten Tabellen und/oder mithilfe eines Bodenmodells, das weitere Parameter (z. B. Bodenarten im Bodenareal BA1, Humusanteil, pH-Wert) berücksichtigt. Der fünfte Schritt S5 kann zum Beispiel mithilfe der Ermittlungseinrichtung 24 und entsprechender Programmroutinen erfolgen.

Fig. 1B zeigt eine schematische Ansicht eines weiteren Ausführungsbeispiels des erfindungsgemäßen Verfahrens. In einem ersten Schritt SW1 wird ein elektromagnetisches Signal SI2 mithilfe einer Sendeeinrichtung, die die Sendeeinrichtung 1 an der ersten Position P1 sein kann, ausgesendet. Die Frequenz des elektromagnetischen Signals SI2 liegt zum Beispiel im Frequenzbereich der Kurzwellen, im Frequenzbereich der Mittelwellen oder im Frequenzbereich der Langwellen. Das elektromagnetische Signal SI2 kann zum Beispiel senderseitig dem elektromagnetischen Signal SI1 entsprechen. Für die weiteren Betrachtungen wird davon ausgegangen, dass das elektromagnetische Signal SI2 dem elektromagnetischen Signal SI1 senderseitig entspricht.

Das elektromagnetische Signal SI2 kann inhaltlich ein Identifikationsmerkmal oder Informationen über den geographischen Ort der ersten Position P1 und/oder Informationen über einen Sendezeitpunkt aufweisen, ähnlich, wie in Bezug auf SI1 beschrieben. Auch Ausführungen zu der ersten Position P1 und zur Sendeantenne 11 gelten für dieses Ausführungsbeispiel entsprechend. Neu ist nun, dass ein anderes Bodenareal, nämlich das Bodenareal BA2 (vgl. Fig. 2B) mithilfe des erfindungsgemäßen Verfahrens untersucht werden soll. Das Bodenareal BA2 ist zwischen der ersten Position P1 und einer weiteren (also zweiten) Position, zum Beispiel der dritten Position P3 (vgl. Fig. 2B) befindlich. Eine Empfangseinrichtung, die zum Beispiel die Empfangseinrichtung 3 (vgl. Fig. 2B) sein kann, befindet sich mit einer Empfangsantenne 31 an der dritten Position P3.

Eine Längsausdehnung des Bodenareals BA2 kann zum Beispiel zwischen 15 km bis 400 km liegen. Auch Längsausdehnungen darüber oder darunter sind nicht ausgeschlossen. Es wird angenommen, dass die Längsausdehnung des Bodenareals BA2 größer ist als die Längsausdehnung des Bodenareals BA1. Es wird angenommen, dass das Bodenareal BA1 als Teilbereich TBA1 (vgl. Fig. 2B) im Bodenareal BA2 enthalten ist wie in Fig. 3 dargestellt. Zusätzlich enthält das Bodenareal BA2 den Teilbereich TBA2 (vgl. Fig. 2B). Der Teilbereich TBA2 weist eine andere Bodenart auf als der Teilbereich TBA1. In Bezug auf die Breite und die Tiefe des Bodenareals BA2 gelten die gleichen Bemerkungen sinngemäß wie in Bezug auf die Breite und die Tiefe des Bodenareals BA1. Auf dem Weg von der Sendeeinrichtung 1 bis zur Empfangseinrichtung 3 liegt der Teilbereich TBA2 nach dem Teilbereich TBA1.

In einem zweiten Schritt SW2 wird das elektromagnetische Signal SI2 empfangen mithilfe der Empfangseinrichtung 3. Die Empfangseinrichtung 3 ist ausgestaltet, das elektromagnetische Signal SI2 als Bodenwelle zu empfangen. Die Empfangseinrichtung 3 kann der Empfangseinrichtung 2 in ihrer Ausgestaltung entsprechen. Die Empfangseinrichtung 3 kann auch die Empfangseinrichtung 2 sein, und zwar zum Beispiel, nachdem die Empfangseinrichtung 2 an die dritte Position P3 verbracht worden ist zwecks Untersuchung des weiteren Teilbereichs TBA2. Bemerkungen zu der zweiten Position P2 können sinngemäß auch für die dritte Position P3 gelten.

Das Bodenareal BA2 weist zwei Teilbereiche auf, den Teilbereich TBA1 und den Teilbereich TBA2. Für den Teilbereich TBA1 gelten die Bemerkungen zum Bodenareal BA1 entsprechend. Der Teilbereich TBA2 weist eine andere Bodenart als der Teilbereich TBA1 - also eine andere Bodenart als das Bodenareal BA1 - auf.

Im zweiten Schritt SW2 können zusätzlich insbesondere ein Empfangszeitpunkt des Signals SI2 und/oder Informationen über den geographischen Ort der dritten Position P3 ermittelt werden. Dies kann zum Beispiel mithilfe eines Empfängers eines globalen Navigationssatellitensystems, zum Beispiel dem empfängerseitigen Empfänger 35 (vgl. Fig. 2B) eines globalen Navigationssatellitensystems erfolgen.

In einem dritten Schritt SW3 wird ein Phasenwert PH2 des elektromagnetischen Signals SI2 ermittelt. Die Ermittlung kann mithilfe einer Ermittlungseinrichtung erfolgen, zum Beispiel der Ermittlungseinrichtung 34 (vgl. Fig. 2B), die der Ermittlungseinrichtung 24 entsprechen kann. Der Phasenwert PH2 kann insbesondere zu einem vordefinierten Zeitpunkt, zum Beispiel einem vordefinierten Zeitpunkt nach Absenden des elektromagnetischen Signals SI2, gemessen werden.

In einem vierten Schritt SW4 wird mithilfe des Phasenwerts PH2 ein Wert EL2 der elektrischen Leitfähigkeit des Bodenareals BA2 ermittelt. Es kann sich zum Beispiel um einen durchschnittlichen Wert beider Teilbereiche TBA1 und TBA2 handeln. Alternativ kann der Wert EL2 der elektrischen Leitfähigkeit derart bestimmt werden, dass er lediglich den Teilbereich TBA2 betrifft. Hierfür kann eine Voraussetzung sein, dass für den Teilbereich TBA1 bereits ein Wert einer Leitfähigkeit oder ein Feuchtigkeitswert (der zum Beispiel als Referenz verwendet werden kann), zum Beispiel der Feuchtigkeitswert F1, oder ein Modell oder Messergebnisse hinsichtlich der Feuchtigkeit im Teilbereich TBA1 bereits vorliegen. Zusätzlich wird vorgeschlagen, auch den Feuchtigkeitswert des zweiten Teilbereichs TBA2, oder alternativ ohne Vorkenntnis des Feuchtigkeitswerts F1 des ersten Teilbereichs TBA1, wie in der allgemeinen Beschreibung detailliert beschrieben auf Basis eines Modells zu ermitteln.

Der Referenzphasenwert RP1 und/oder weitere Referenzphasenwerte können im vierten Schritt SW4 hinzugezogen werden, zum Beispiel ein Referenzphasenwert RP2. Der Wert EL2 kann zum Beispiel durch Vergleich oder Abgleich mit dem Referenzphasenwert RP1 und dem Referenzphasenwert RP2 ermittelt werden.

Der Referenzphasenwert RP2 kann zum Beispiel unter Berücksichtigung
- des Referenzphasenwerts RP1 (der einen Einfluss des Teilbereichs TBA1 und dessen Bodenart beschreibt) und/oder
- der Längsausdehnung des Bodenareals BA2 und/oder
- einer bekannten (zum Beispiel experimentell ermittelten) Signalausbreitungsgeschwindigkeit
   - in einer bestimmten Bodenart und/oder
   - bei einem bekanntem (zum Beispiel experimentell ermittelten) Wert der elektrischen Leitfähigkeit und/oder
   - bei einer Frequenz, die der Frequenz des elektromagnetischen Signals SI2 entspricht,
rechnerisch ermittelt worden sein, wobei die Bodenart der Bodenart des Teilbereichs TBA2 entsprechen kann.

Der Referenzphasenwert RP2 kann auch zum Beispiel unter Nutzung des vorgestellten Systems ermittelt, insbesondere gemessen worden sein. Der Referenzphasenwert kann insbesondere zum Beispiel der bereits ermittelte Phasenwert PH1 sein. Dies könnte zum Beispiel ermöglichen, den Einfluss des Teilbereichs TBA1 auf den Phasenwert PH2 zu quantifizieren (zum Beispiel herausrechnen oder bestimmen) zu können, um dann den Einfluss des Teilbereichs TBA2 und dessen elektrische Leitfähigkeit separat quantifizieren zu können. Der Wert EL2 der elektrischen Leitfähigkeit kann sich dann wie bereits erwähnt alleine auf den Teilbereich TBA2 beziehen.

In einem fünften Schritt SW5 wird anhand des Wertes EL2 der elektrischen Leitfähigkeit mittels bekannter Zusammenhänge, die insbesondere durch das Modell berücksichtigt werden, ein Feuchtigkeitswert F2 der durchschnittlichen Bodenfeuchtigkeit des Bodenareals BA2 ermittelt, zum Beispiel anhand von bekannten Tabellen. Der fünfte Schritt SW5 kann zum Beispiel mithilfe der Ermittlungseinrichtung 34 und entsprechender Programmroutinen erfolgen. Alternativ kann der Feuchtigkeitswert F2 im Rahmen des fünften Schritts SW5 - insbesondere, wenn der Wert EL2 sich alleine auf den Teilbereich TBA2 bezieht - derart ermittelt werden, dass er sich nur auf den Teilbereich TBA2 bezieht. Der Feuchtigkeitswert wird auf Basis des Modells des Bodenareals ermittelt. Insbesondere kann die Bodenfeuchtigkeit der Teilbereiche TBA1 und TBA2 jeweils eine Variable des Modells sein.

In Fig. 2A wird eine schematische Ansicht eines erfindungsgemäßen Systems und eines zu untersuchenden Bodenareals gezeigt. im linken Bereich ist die erste Position P1 anhand eines Kreuzes angezeigt. An der ersten Position P1 befindet sich die Sendeantenne 11, angezeigt durch einen Punkt im Mittelpunkt des Kreuzes. Anhand von Fig. 3, die den senderseitigen Teil der Fig. 2A detaillierter zeigt, wird schematisch gezeigt, dass sich die Sendeantenne 11 einerseits oberhalb des Bodens B befindet und andererseits unterhalb durch ein grobmaschiges Netz, das in den Boden B eingelassen ist zum Beispiel in einer Tiefe von ca. 50 cm bis 1 Meter, mit dem Boden B leitend verbunden ist. Das heißt, die Sendeantenne 11 ist teilweise im Boden B befindlich und mit diesem elektrisch verbunden. Die Sendeeinrichtung 1 weist neben der Sendeantenne 11 weitere Bestandteile 12, zum Beispiel einen Oszillator und einen Verstärker auf. Mithilfe dieser weiteren Bestandteile 12 können elektromagnetische Signale generiert werden. Zudem ist eine Steuereinrichtung 14 vorgesehen. Die Sendeantenne 11 ist mit den weiteren Bestandteilen über eine elektrische Verbindung, die als Linie dargestellt wird, verbunden. Die Steuereinrichtung 14, die zum Beispiel zum Einstellen einer Frequenz oder zum Gestalten auszusendender elektromagnetischer Signale, zum Beispiel dem elektromagnetischen Signal SI1, ausgestaltet sein kann, ist ebenfalls über eine elektrische Leitung verbunden.

Sowohl mit der Steuereinrichtung 14 als auch mit den weiteren Bestandteilen 12 ist der senderseitige Empfänger 15 eines globalen Navigationssatellitensystems verbunden, der insbesondere ausgestaltet sein kann, Informationen über einen Sendezeitpunkt zwecks Synchronisierung, zum Beispiel mit der Empfangseinrichtung 2, und/oder Informationen über einen geographischen Ort der Position P1, zum Beispiel zwecks genauer Bestimmung der Distanz zu der Empfangseinrichtung 2, zu ermitteln. Alternativ kann anstelle des senderseitigen Empfängers 15 eines globalen Navigationssatellitensystems auch eine andere Positionsbestimmungseinrichtung (z. B. Empfänger eines anderen, z. B. terrestrischen Navigationssystems) und eine andere Zeitbestimmungseinrichtung (z. B. Empfänger eines Mobilfunksignals, das Zeitinformationen enthält) vorgesehen sein. Zur Positionsbestimmung wäre auch eine geodätische Einmessung ausreichend.

Das Bodenareal BA1 befindet sich zwischen der Position P1 und der Position P2. An der Position P2 befindet sich die Empfangsantenne 21 als Teil der Empfangseinrichtung 2. Anhand von Fig. 4, die den empfängerseitigen Teil der Fig. 2A detaillierter zeigt, wird schematisch gezeigt, dass sich die Empfangsantenne 21 am und oberhalb des Bodens B befindet. Die Empfangseinrichtung 2 kann portabel ausgestaltet sein.

Die Empfangseinrichtung 2 ist über eine elektrische Verbindung mit weiteren Bestandteilen 22 der Empfangseinrichtung 2 verbunden. Die weitere Bestandteile 22 können zum Beispiel einen Filter und einen Verstärker umfassen.

Zudem ist die Ermittlungseinrichtung 24 vorgesehen. Die Ermittlungseinrichtung 24 dient zum Ermitteln von Werten einer physikalischen Eigenschaft von empfangenen elektromagnetischen Signalen, insbesondere einer Phase. Im vorliegenden Fall kann die Ermittlungseinrichtung 24 insbesondere zum Beispiel ausgestaltet sein, die Phase PH1 zu ermitteln. Sie kann auch zusätzlich ausgestaltet sein, Werte der elektrischen Leitfähigkeit, insbesondere den Wert EL1 der elektrischen Leitfähigkeit des Bodenareals BA1, zu ermitteln. Sie kann auch zusätzlich ausgestaltet sein, Feuchtigkeitswerte, insbesondere den Feuchtigkeitswert F1 des Bodenareals BA1, zu ermitteln. Die Ermittlungseinrichtung 24 kann eine Recheneinheit oder mehrere Recheneinheiten, wobei eine Recheneinheit insbesondere einen Computer mit CPU und entsprechender Software aufweisen kann, umfassen. Mindestens eine Recheneinheit der Ermittlungseinrichtung kann an einem von der Empfangseinrich-tung 2 entfernten Ort, zum Beispiel in einem Gebäude, angeordnet sein. Die Ermittlungseinrichtung 24 ist datentechnisch (zum Beispiel drahtgebunden oder drahtlos, gegebenenfalls auch über eine weitere Distanz) mit den weiteren Bestandteilen 22 verbunden.

Sowohl mit der Ermittlungseinrichtung 24 als auch mit den weiteren Bestandteilen 22 ist der empfängerseitige Empfänger 25 eines globalen Navigationssatellitensystems datentechnisch verbunden, der insbesondere ausgestaltet sein kann, Informationen über einen Empfangszeitpunkt zwecks Synchronisierung, zum Beispiel mit der Sendeeinrichtung 1, und/oder Informationen über einen geographischen Ort der Position P2, zum Beispiel zwecks genauer Bestimmung der Distanz zu der Sendeeinrichtung 1, zu ermitteln. Alternativ kann anstelle des empfängerseitigen Empfängers 25 eines globalen Navigationssatellitensystems auch eine andere Positionsbestimmungseinrichtung (z. B. Empfänger eines anderen, z. B. terrestrischen Navigationssystems) und eine andere Zeitbestimmungseinrichtung (z. B. Empfänger eines Mobilfunksignals, das Zeitinformationen enthält) vorgesehen sein. Bei der Ausgestaltung als statischer Empfänger wäre auch eine geodätische Einmessung zur Positionsbestimmung ausreichend.

In Fig. 2B werden die bereits vorgestellten empfangsseitigen Einheiten gezeigt sowie die dritte Position P3, die Empfangsantenne 31, weitere Bestandteile 32, die Ermittlungseinrichtung 34 und den empfängerseitigen Empfänger 35 eines globalen Navigationssatellitensystems gezeigt. Die Bemerkungen hinsichtlich der Empfangsantenne 21, den weiteren Bestandteilen 22, der Ermittlungseinrichtung 24 und des empfängerseitigen Empfängers 25 eines globalen Navigationssatellitensystems (und die Bemerkungen hinsichtlich dessen Alternativen) gelten entsprechend.

In Bezug auf die vorgestellten Ausgestaltungen des erfindungsgemäßen Systems wird vollinhaltlich auf die Bemerkungen zu den vorgestellten Ausgestaltungen des erfindungsgemäßen Verfahrens - und umgekehrt - Bezug genommen.

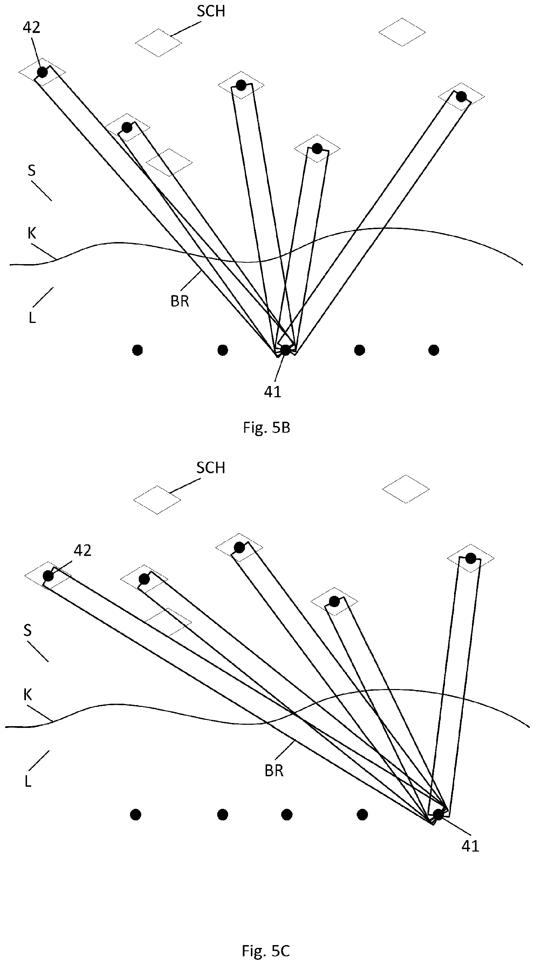

In den Fig. 5A bis 5C werden jeweils ein Landabschnitt L, ein Seeabschnitt S, ein Küstenstreifen K sowie verschiedene auf dem Seeabschnitt S befindliche Schiffe SCH gezeigt. Die Schiffe SCH werden als rautenförmige Symbole dargestellt. Manche der Schiffe SCH weisen Empfangseinrichtungen 42 auf, die als kleiner Kreis markiert sind. Die Schiffe SCH bewegen sich im zeitlichen Verlauf auf dem Seeabschnitt S. Der Übersichtlichkeit halber werden die Bezugszeichen "42" und "SCH" auf den Figuren 5A bis 5C jeweils nur einfach gezeigt. Weiterhin zeigen die Fig. 5A bis 5C Sendeeinrichtungen 41, die sich an fünf verschiedenen Positionen befinden. Das Bezugszeichen "41" wird aus Gründen der Übersichtlichkeit ebenfalls jeweils nur einmal gezeigt. In den Figuren finden sich weiterhin Bodenareale BR, die schematisch durch langgezogene Rechtecke dargestellt sind. Die Bodenareale BR erstrecken sich jeweils in Längsrichtung von einer der Sendeeinrichtungen 41 über einen Küstenstreifen K hinweg bis zu einer der Empfangseinrichtungen 42. Die Bodenareale BR weisen jeweils sowohl eine Breite als auch eine Tiefe auf, die nicht näher bestimmt zu werden braucht. Die Bodenareale BR weisen jeweils einen Teilbereich in einem Landabschnitt L und einen Teilbereich in einem Seeabschnitt S auf.

Mithilfe der Sendeeinrichtungen 41 und den Empfangseinrichtungen 42 ist das erfindungsgemäße Verfahren für die gezeigten Bodenareale BR durchführbar.

Bedeutsam ist, dass auf diese Art und Weise große Landabschnitte L untersucht werden können, die sich von den in den Fig. 5A-5C gezeigten Sendeeinrichtungen 41 bis hin zu den jeweiligen Schnittbereichen mit dem Küstenstreifen K erstrecken. Unter Nutzung mehrerer Sendeeinrichtungen 41 und mehrerer Empfangseinrichtungen 42, wie in den Fig. 5A-5C gezeigt, kann ein detaillierter Plan von Verteilungen von Bodenfeuchtigkeitswerten der Landabschnitte L aufgestellt werden.

Hierbei kann es erforderlich sein, dass die genutzten elektromagnetischen Signale Identifikationsmerkmale aufweisen, um die elektromagnetischen Signale anhand verschiedener Sendeeinrichtungen 41 unterscheiden zu können. Einflüsse der Seeabschnitte S können herausgerechnet werden, da die Ausbreitungseigenschaften elektromagnetischer Wellen an der Wasseroberfläche bekannt sind.

### Bezugszeichenliste:

- 1: Sendeeinrichtung
- 11: Sendeantenne
- 12: weitere Bestandteile der Sendeeinrichtung
- 14: Steuereinrichtung
- 15: senderseitiger Empfänger eines globalen Navigationssatellitensystems
- 2: Empfangseinrichtung
- 21: Empfangsantenne
- 22: weitere Bestandteile der Empfangseinrichtung
- 24: Ermittlungseinrichtung
- 25: empfängerseitiger Empfänger eines globalen Navigationssatellitensystems
- 3: Empfangseinrichtung
- 31: Empfangsantenne
- 32: weitere Bestandteile der Empfangseinrichtung
- 34: Ermittlungseinrichtung
- 35: empfängerseitiger Empfänger eines globalen Navigationssatellitensystems
- 41: Sendeeinrichtung
- 42: Empfangseinrichtung
- B: Boden
- BA1: Bodenareal
- BA2: Bodenareal
- BR: Bodenareal
- EL1: Wert der elektrischen Leitfähigkeit
- EL2: Wert der elektrischen Leitfähigkeit
- F1: Feuchtigkeitswert
- F2: Feuchtigkeitswert
- K: Küstenstreifen
- L: Landabschnitt
- P1: erste Position
- P2: zweite Position
- P3: dritte Position
- PH1: Phasenwert
- PH2: Phasenwert
- RP1: Referenzphasenwert
- RP2: Referenzphasenwert
- S: Seeabschnitt
- S1: erster Schritt
- S2: zweiter Schritt
- S3: dritter Schritt
- S4: vierter Schritt
- S5: fünfter Schritt
- SCH: Schiff
- SI1: elektromagnetisches Signal
- SI2: elektromagnetisches Signal
- SW1: erster Schritt
- SW2: zweiter Schritt
- SW3: dritter Schritt
- SW4: vierter Schritt
- SW5: fünfter Schritt
- TBA1: Teilbereich 1 eines Bodenareals
- TBA2: Teilbereich 2 eines Bodenareals

## Patentansprüche

1. Verfahren zur Ermittlung von Feuchtigkeitswerten (F1; F2) eines Bodenareals (BA2; BR), das sich zwischen einer ersten Position (P1) und einer zweiten Position (P3) befindet,
aufweisend:
- Senden eines elektromagnetischen Signals (SI2) an der ersten Position (P1) mithilfe einer Sendeeinrichtung (1; 41) derart, dass es sich als Bodenwelle verbreitet;
- Empfangen des elektromagnetischen Signals (SI2) an der zweiten Position (P2) mithilfe einer Empfangseinrichtung (3; 42); **gekennzeichnet durch**:
- Ermitteln je eines Wertes von zumindest zwei physikalischen Eigenschaften des elektromagnetischen Signals (SI2), nämlich eines Phasenwertes (PH1, PH2) des empfangenen elektromagnetischen Signals und eines Wertes einer Amplitude eines Feldes des elektromagnetischen Signals;
- Ermitteln von Feuchtigkeitswerten (F1; F2) des Bodenareals (BA2; BR) mithilfe der Werte der physikalischen Eigenschaften;
wobei
- das Bodenareal (BA2; BR) eine Mehrzahl von Teilbereichen (TBA1, TBA2) aufweist, die bezüglich einer entlang der Bodenoberfläche des Bodenareals (BA2; BR) verlaufenden direkten Verbindungslinie der ersten Position (P1) mit der zweiten Position (P3) hintereinander liegend angeordnet sind,
- basierend auf einem Modell des Bodenareals (BA2; BR), welches jeweils zumindest eine elektrische physikalische Größe der Teilbereiche (TBA1, TBA2) in Abhängigkeit von den zumindest zwei physikalischen Eigenschaften des elektromagnetischen Signals (SI2) an der zweiten Position beschreibt, jeweils der Feuchtigkeitswert (F1; F2) der Mehrzahl der Teilbereiche (TBA1, TBA2) des Bodenareals (BA2; BR) ermittelt wird,
wobei auf Basis des Modells für die Teilbereiche (TBA1, TBA2) unter Berücksichtigung von Eigenschaften des jeweiligen Bodens in den Teilbereichen (TBA1, TBA2) jeweils ein Startwert der zumindest einen elektrischen physikalischen Größe für jeden der Teilbereiche (TBA1, TBA2) ermittelt wird,
wobei das Modell unter Veränderung der Startwerte an die ermittelten Werte der zumindest zwei physikalischen Eigenschaften des an der zweiten Position (P2) empfangenen elektromagnetischen Signals (SI2) angepasst wird und
wobei die Feuchtigkeitswerte (F1; F2) der Mehrzahl der Teilbereiche (TBA1, TBA2) des Bodenareals (BA2; BR) basierend auf dem angepassten Modell ermittelt werden.

2. Verfahren gemäß Anspruch 1, **gekennzeichnet dadurch, dass** bei dem Ermitteln des Feuchtigkeitswerts (F1; F2) zusätzlich ein Referenzwert (RP1, RP2) genutzt wird.

3. Verfahren gemäß einem der Ansprüche 1-2, **gekennzeichnet dadurch, dass** die physikalische Eigenschaft des elektromagnetischen Signals (SI2) auch eine Laufzeit des elektromagnetischen Signals (SI2) umfasst.

4. Verfahren gemäß einem der Ansprüche 1-3, **gekennzeichnet dadurch, dass** mithilfe des Wertes der physikalischen Eigenschaft des elektromagnetischen Signals (SI2) ein Wert einer elektrischen physikalischen Größe des jeweiligen Teilbereichs (TBA1, TBA2) des Bodenareals (BA2; BR) ermittelt wird und der Feuchtigkeitswert (F1; F2) mithilfe des Wertes der elektrischen physikalischen Größe ermittelt wird.

5. Verfahren gemäß Anspruch 4, **gekennzeichnet dadurch, dass** die elektrische physikalische Größe eine elektrische Leitfähigkeit (EL1; EL2) oder eine Impedanz oder eine dielektrische Eigenschaft des jeweiligen Teilbereichs (TBA1, TBA2) des Bodenareals (BA2; BR) ist.

6. Verfahren gemäß einem der Ansprüche 4-5, **gekennzeichnet dadurch, dass** der Wert der elektrischen physikalischen Größe des jeweiligen Teilbereichs (TBA1, TBA2) des Bodenareals (BA2; BR) zusätzlich mithilfe von Bodendaten und/oder Bodenkarten des Bodenareals (BA2; BR) ermittelt wird.

7. Verfahren gemäß einem der Ansprüche 4-6, **gekennzeichnet dadurch**, der Wert der elektrischen physikalischen Größe des jeweiligen Teilbereichs (TBA1, TBA2) des Bodenareals (BA2; BR) zusätzlich mithilfe eines Werts einer Temperatur des jeweiligen Teilbereichs (TBA1, TBA2) des Bodenareals (BA2; BR) ermittelt wird.

8. Verfahren gemäß einem der Ansprüche 1-7, **gekennzeichnet dadurch, dass** die Anpassung des Modells iterativ durch wiederholte Veränderung der Werte der zumindest einen elektrischen physikalischen Größe erfolgt und das die Iteration beendet wird, wenn ein Abbruchkriterium erfüllt ist.

9. Verfahren gemäß einem der Ansprüche 1-8, **gekennzeichnet dadurch, dass** die Eigenschaften des jeweiligen Bodens in den Teilbereichen (TBA1, TBA2) zumindest eine zustandsunabhängige Eigenschaft und zumindest eine zustandsabhängige Eigenschaft aufweisen, wobei zustandsunabhängige Eigenschaften solche Eigenschaften sind, die sich aus einer Beschreibung der Bodenart in dem Teilbereich ergeben, und wobei zustandsabhängige Eigenschaften den Zustand des Bodens beschreiben, beispielsweise die Temperatur und die Bodenfeuchte.

10. Verfahren gemäß einem der Ansprüche 1-9 **gekennzeichnet dadurch, dass** ein Wert einer Energiedichte des elektromagnetischen Signals (SI2) und/oder ein Wert einer Leistung des elektromagnetischen Signals (SI2) ermittelt wird/werden, und der Feuchtigkeitswert (F1; F2) des Bodenareals (BA2; BR) zusätzlich mithilfe des Wertes der Energiedichte und/oder des Wertes der Leistung ermittelt wird.

11. Verfahren gemäß einem der Ansprüche 1-10, **gekennzeichnet dadurch, dass** eine Mehrzahl von Sendeeinrichtungen (1; 41) an verschiedenen Standorten und/oder eine Mehrzahl von Empfangseinrichtungen (3; 42) an verschiedenen Standorten vorgesehen ist und das Verfahren mithilfe der Mehrzahl von Sendeeinrichtungen (1; 41) und/oder der Mehrzahl von Empfangseinrichtungen (3; 42) für eine Mehrzahl von Bodenarealen (BA2; BR) durchgeführt wird.

12. Verfahren gemäß einem der Ansprüche 1-11, **gekennzeichnet dadurch, dass**
- die Frequenz des elektromagnetischen Signals zu einer weiteren Frequenz verändert wird und
- mindestens ein weiterer Wert der physikalischen Eigenschaft des elektromagnetischen Signals bei der weiteren Frequenz ermittelt wird und
- mithilfe des weiteren Werts der physikalischen Eigenschaft ein weiterer Feuchtigkeitswert des Bodenareals (BA2; BR) ermittelt wird.

13. System zur Durchführung des Verfahrens zur Ermittlung von Feuchtigkeitswerten (F1; F2) eines Bodenareals (BA2; BR) gemäß einem der Ansprüche 1-12 aufweisend
- eine Sendeeinrichtung (1; 41), die ausgestaltet ist, ein elektromagnetisches Signal (SI2) derart zu senden, dass es sich als Bodenwelle verbreitet;
- eine Empfangseinrichtung (3; 42), die ausgestaltet ist, das elektromagnetische Signal (SI2) zu empfangen; **gekennzeichnet durch**:
- eine Ermittlungseinrichtung (24; 34), die ausgestaltet ist, je einen Wert von zumindest zwei physikalischen Eigenschaften des elektromagnetischen Signals (SI2) zu ermitteln, nämlich einen Phasenwert (PH1, PH2) des empfangenen elektromagnetischen Signals und einen Wert einer Amplitude eines Feldes des elektromagnetischen Signals;
wobei die Ermittlungseinrichtung (24; 34) zusätzlich ausgestaltet ist, basierend auf einem Modell des Bodenareals (BA2; BR), welches jeweils zumindest eine elektrische physikalische Größe der Teilbereiche (TBA1, TBA2) in Abhängigkeit von den zumindest zwei physikalischen Eigenschaften des elektromagnetischen Signals (SI2) an der zweiten Position beschreibt, Feuchtigkeitswerte (F1; F2) einer Mehrzahl von Teilbereichen (TBA1, TBA2) des Bodenareals (BA2; BR),
wobei auf Basis des Modells für die Teilbereiche (TBA1, TBA2) unter Berücksichtigung von Eigenschaften des jeweiligen Bodens in den Teilbereichen (TBA1, TBA2) jeweils ein Startwert der zumindest einen elektrischen physikalischen Größe für jeden der Teilbereiche (TBA1, TBA2) ermittelt wird,
wobei das Modell unter Veränderung der Startwerte an die ermittelten Werte der zumindest zwei physikalischen Eigenschaften des an der zweiten Position (P2) empfangenen elektromagnetischen Signals (SI2) angepasst wird und
wobei die Feuchtigkeitswerte (F1; F2) der Mehrzahl der Teilbereiche (TBA1, TBA2) des Bodenareals (BA2; BR) basierend auf dem angepassten Modell ermittelt wird.

14. System gemäß Anspruch 13, wobei die Sendeeinrichtung und/oder die Empfangseinrichtung (2; 3; 42) eine Positionsbestimmungsvorrichtung und/oder eine Zeitbestimmungsvorrichtung aufweist/aufweisen, insbesondere einen Empfänger eines globalen Navigationssatellitensystems (15; 25; 35).

## Claims

1. A method for determining moisture values (F1; F2) in a ground area (BA2; BR), which is located between a first position (P1) and a second position (P3),
comprising:
- sending an electromagnetic signal (SI2) at the first position (P1) by means of a transmitting device (1; 41) such that it propagates as a ground wave;
- receiving the electromagnetic signal (SI2) at the second position (P2) by means of a receiving device (3; 42), **characterized by**
- determining a value of at least two physical properties of the electromagnetic signal (SI2), namely a phase value (PH1; PH2) of the received electromagnetic signal (SI2), and a value of an amplitude of a field of the electromagnetic signal;
- determining moisture values (F1; F2) of the ground area (BA2; BR) using the values of the physical properties; wherein
- the ground area (BA2; BR) has a plurality of subareas (TBA1, TBA2), which are arranged one behind the other with respect to a direct connecting line of the first position (P1) with the second position (P3) extending along the ground surface of the ground area (BA2; BR),
- based on a model of the ground area (BA2; BR), which describes at least one electrical physical variable of the subareas (TBA1, TBA2) as a function of the at least two physical properties of the electromagnetic signal (SI2) at the second position, the moisture value (F1; F2) of the plurality of subareas (TBA1, TBA2) of the ground area (BA2; BR) is determined respectively,
wherein
on the basis of the model for the subareas (TBA1, TBA2) an initial value of the at least one electrical physical variable for each of the subareas (TBA1, TBA2) is determined respectively, taking into consideration properties of the respective soil in the subareas (TBA1, TBA2),
wherein the model is adapted to the determined values of the at least two physical properties of the electromagnetic signal (SI2) received in the second position (P2) by changing the initial values, and
wherein the moisture values (F1; F2) of the plurality of subareas (TBA1, TBA2) of the ground area (BA2; BR) are determined based on the adapted model.

2. The method according to claim 1, **characterized in that** a reference value (RP1, RP2) is additionally used when determining the moisture value (F1; F2).

3. The method according to any one of claims 1-2, **characterized in that** the physical property of the electromagnetic signal (SI2) also comprises a transit time of the electromagnetic signal (SI2).

4. The method according to any one of claims 1-3, **characterized in that** by means of the value of the physical property of the electromagnetic signal (SI2) a value of an electrical physical variable of the respective subarea (TBA1, TBA2) of the ground area (BA2; BR) is determined and the moisture value (F1; F2) is determined by means of the value of the electrical physical variable.

5. The method according to claim 4, **characterized in that** the electrical physical variable is an electrical conductivity (EL1; EL2) or an impedance or a dielectric property of the respective subarea (TBA1, TBA2) of the ground area (BA2; BR).

6. The method according to any one of claims 4-5, **characterized in that** the value of the electrical physical variable of the respective subarea (TBA1, TBA2) of the ground area (BA2; BR) is additionally determined by means of soil data and/or soil maps of the ground area (BA2; BR).

7. The method according to any one of claims 4-6, **characterized in that** the value of the electrical physical variable of the respective subarea (TBA1, TBA2) of the ground area (BA2; BR) is additionally determined by means of a value of a temperature of the respective subarea (TBA1, TBA2) of the ground area (BA2; BR).

8. The method according to any one of claims 1-7, **characterized in that** the adaptation of the model is performed iteratively by repeatedly changing the values of the at least one electrical physical variable and **in that** the iteration is ended when a termination criterion is met.

9. The method according to any one of claims 1-8, **characterized in that** the properties of the respective soil in the subareas (TBA1, TBA2) have at least one state-independent property and at least one state-dependent property, wherein state-independent properties are properties which are the result of a description of the type of soil in the subarea, and wherein state-dependent properties describe the state of the soil, for example the temperature and the soil moisture.

10. The method according to any one of claims 1-9, **characterized in that** a value of an energy density of the electromagnetic signal (SI2) and/or a value of a power of the electromagnetic signal (SI2) is/are determined, and the moisture value (F1; F2) of the ground area (BA2; BR) is additionally determined by means of the value of the energy density and/or the value of the power.

11. The method according to any one of claims 1-10, **characterized in that** a plurality of transmitting devices (1; 41) are provided at different locations and/or a plurality of receiving devices (3; 42) are provided at different locations and the method is performed using the plurality of transmitting devices (1; 41) and/or the plurality of receiving devices (3; 42) for a plurality of ground areas (BA2; BR).

12. The method according to any one of claims 1-11, **characterized in that**
- the frequency of the electromagnetic signal is changed to a further frequency and
- at least one further value of the physical property of the electromagnetic signal is determined at the further frequency and
- a further moisture value of the ground area (BA2; BR) is determined by means of the further value of the physical property.

13. A system for performing the method for determining moisture values (F1; F2) of a ground area (BA2; BR) according to any one of claims 1-12, comprising:
- a transmitting device (1; 41), which is configured to transmit an electromagnetic signal (SI2) such that it propagates as a ground wave;
- a receiving device (3; 42), which is configured to receive the electromagnetic signal (SI2), **characterized by**
- a determining device (24; 34), which is configured to determine a value of at least two physical properties of the electromagnetic signal (SI2), namely a phase value (PH1; PH2) of the received electromagnetic signal (SI2) and a value of an amplitude of a field of the electromagnetic signal;
wherein the determining device (24; 34) is additionally configured, based on a model of the ground area (BA2; BR), which describes at least one electrical physical variable of the subareas (TBA1, TBA2) as a function of the at least two physical properties of the electromagnetic signal (SI2) at the second position, moisture values (F1; F2) of a plurality of subareas (TBA1, TBA2) of the ground area (BA2; BR),
wherein on the basis of the model for the subareas (TBA1, TBA2) an initial value of the at least one electrical physical variable for each of the subareas (TBA1, TBA2) is determined respectively, taking into consideration properties of the respective soil in the subareas (TBA1, TBA2),
wherein the model is adapted to the determined values of the at least two physical properties of the electromagnetic signal (SI2) received in the second position (P2) by changing the initial values and
wherein the moisture values (F1; F2) of the plurality of subareas (TBA1, TBA2) of the ground area (BA2; BR) is determined based on the adapted model.

14. The system according to claim 13, wherein the transmitting device and/or the receiving device (2; 3; 42) has/have a position determining device and/or a time determining device, in particular a receiver of a global navigation satellite system (15; 25; 35).

## Revendications

1. Procédé de détermination de valeurs d'humidité (F1 ; F2) d'une surface de sol (BA2 ; BR) qui se situe entre une première position (P1) et une deuxième position (P3)
présentant :
- l'envoi d'un signal électromagnétique (SI2) à la première position (P1) à l'aide d'un dispositif émetteur (1 ; 41) de telle manière qu'il se propage sous forme d'une onde de sol ;
- la réception du signal électromagnétique (SI2) à une deuxième position (P2) à l'aide d'un dispositif récepteur (3 ; 42) ;
**caractérisé par** :
- la détermination chaque fois d'une valeur d'au moins deux caractéristiques physiques du signal électromagnétique (SI2), à savoir d'une valeur de la phase (PH1, PH2) du signal électromagnétique (SI2) réceptionné et d'une valeur d'une amplitude d'un champ du signal électromagnétique ;
- la détermination de valeurs d'humidité (F1 ; F2) de la surface de sol (BA2 ; BR) à l'aide des valeurs des caractéristiques physiques ;
dans lequel
- la surface de sol (BA2 ; BR) présente une multiplicité de zones partielles (TBA1, TBA2) qui sont disposées se situant les unes derrière les autres par rapport à une ligne de liaison directe de la première position (P1) avec la deuxième position (P3) s'étendant le long d'une surface supérieure de sol de la surface de sol (BA2 ; BR),
- en se basant sur un modèle de la surface de sol (BA2 ; BR), lequel décrit respectivement au moins une grandeur physique électrique des zones partielles (TBA1, TBA2) en fonction des au moins deux propriétés physiques du signal électromagnétique (SI2) à la deuxième position, on peut respectivement déterminer la valeur d'humidité (F1 ; F2) de la multiplicité des zones partielles (TBA1, TBA2) de la surface de sol (BA2 ; BR),
dans lequel, sur la base du modèle pour les zones partielles (TBA1, TBA2), en tenant compte de caractéristiques du sol respectif dans les zones partielles (TBA1, TBA2), on détermine respectivement une valeur de départ d'une grandeur physique électrique pour chacune des zones partielles (TBA1, TBA2),
dans lequel le modèle est adapté, moyennant la modification des valeurs de départ pour les valeurs déterminées des au moins deux caractéristiques physiques du signal électromagnétique (SI2) réceptionné à la deuxième position (P2), et
dans lequel les valeurs d'humidité (F1 ; F2) de la multiplicité des zones partielles (TBA1, TBA2) de la zone de sol (BA2 ; BR) sont déterminées en se basant sur le modèle adapté.

2. Procédé selon la revendication 1, **caractérisé en ce que**, lors de la détermination de la valeur d'humidité (F1 ; F2), on utilise de manière complémentaire une valeur de référence (RP1, RP2).

3. Procédé selon l'une des revendications 1 à 2, **caractérisé en ce que** la caractéristique physique du signal électromagnétique (SI2) comprend également un temps de vol du signal électromagnétique (SI2).

4. Procédé selon l'une des revendications 1 à 3, **caractérisé en ce qu'**avec l'aide de la valeur de la caractéristique physique du signal électromagnétique (SI2), on détermine une valeur d'une grandeur physique électrique de la zone partielle (TBA1, TBA2) respective de la surface de sol (BA2 ; BR) et la valeur de l'humidité (F1 ; F2) est déterminée à l'aide de la valeur de la grandeur physique électrique.

5. Procédé selon la revendication 4, **caractérisé en ce que** la grandeur physique électrique est une conductivité électrique (EL1 ; EL2), ou une impédance, ou une propriété diélectrique de la zone partielle (TBA1, TBA2) respective de la surface de sol (BA2 ; BR).

6. Procédé selon l'une des revendications 4 à 5, **caractérisé en ce que** la valeur de la grandeur physique électrique de la zone partielle (TBA1, TBA2) respective de la surface de sol (BA2 ; BR) est déterminée de manière complémentaire à l'aide de données du sol, et/ou de cartes du sol de la surface de sol (BA2 ; BR).

7. Procédé selon l'une des revendications 4 à 6, **caractérisé en ce que** la valeur de la grandeur physique électrique de la zone partielle (TBA1, TBA2) respective de la surface de sol (BA2 ; BR) est déterminée de manière complémentaire à l'aide d'une température de la zone partielle (TBA1, TBA2) respective de la surface de sol (BA2 ; BR).

8. Procédé selon l'une des revendications 1 à 7, **caractérisé en ce que** l'adaptation du modèle a lieu de manière itérative par la modification répétée des valeurs de l'au moins une grandeur physique électrique et que l'itération est terminée lorsqu'un critère d'interruption est réalisé.

9. Procédé selon l'une des revendications 1 à 8, **caractérisé en ce que** les caractéristiques du sol respectif dans les zones partielles (TBA1, TBA2) présentent au moins une caractéristique indépendante de l'état et au moins une caractéristique dépendante de l'état, où les caractéristiques indépendantes de l'état sont des caractéristiques qui se produisent à partir d'une description du type de sol dans la zone partielle et où les caractéristiques dépendantes de l'état du sol décrivent, par exemple, la température et l'humidité du sol.

10. Procédé selon l'une des revendications 1 à 9, **caractérisé en ce qu'**une valeur de densité d'énergie du signal électromagnétique (SI2) et/ou une valeur d'une puissance du signal électromagnétique (SI2) est/sont déterminée/s et la valeur d'humidité (F1 ; F2) de la surface de sol (BA2 ; BR) est déterminée de manière complémentaire à l'aide de la valeur de la densité d'énergie et/ou de la valeur de la puissance.

11. Procédé selon l'une des revendications 1 à 10, **caractérisé en ce qu'**une multiplicité de dispositifs émetteurs (1 ; 41) est prévue à différents endroits et le procédé est exécuté à l'aide de la multiplicité des dispositifs émetteurs (1 ; 41) et/ou de la multiplicité des dispositifs récepteurs (3 ; 42) pour une multiplicité de surfaces de sol (BA2 ; BR).

12. Procédé selon l'une des revendications 1 à 11, **caractérisé en ce que**
- la fréquence du signal électromagnétique est modifiée pour une nouvelle fréquence et
- au moins une nouvelle valeur de la caractéristique physique du signal électromagnétique est déterminée à la nouvelle fréquence et
- à l'aide de la nouvelle valeur de la caractéristique physique, une nouvelle valeur d'humidité de la surface de sol (BA2 ; BR) est déterminée.

13. Système d'exécution du procédé de détermination de valeurs d'humidité (F1 ; F2) d'une surface-de sol (BA2 ; BR) selon l'une des revendications 1 à 12, présentant
- un dispositif émetteur (1 ; 41) qui est conçu pour émettre un signal électromagnétique (SI2) de telle manière qu'il se propage sous forme d'onde de sol ;
- un dispositif récepteur (3 ; 42) qui est conçu pour réceptionner le signal électromagnétique (SI2) ;
**caractérisé par** :
- un dispositif de détermination (24 ; 34) qui est conçu pour déterminer chaque fois une valeur d'au moins deux caractéristiques du signal électromagnétique (SI2), à savoir une valeur de phase (PH1, PH2) du signal électromagnétique (SI2) réceptionné et une valeur d'une amplitude d'un champ du signal électromagnétique ;
dans lequel le dispositif de détermination (24 ; 34) est en outre conçu, en se basant sur un modèle de la surface de sol (BA2 ; BR), lequel décrit respectivement au moins une grandeur physique électrique des zones partielles (TBA1, TBA2) en fonction des au moins deux caractéristiques physiques du signal électromagnétique (SI2) à la deuxième position, es valeurs d'humidité (f1 ; F2) d'une multiplicité de zones partielles (TBA1, TBA2) de la surface de sol (BA2 ; BR),
dans lequel, sur la base du modèle pour les zones partielles (TBA1, TBA2), en tenant compte de caractéristiques du sol respectif dans les zones partielles (TBA1, TBA2), on détermine respectivement une valeur de départ de l'au moins une grandeur physique électrique pour chacune des zones partielles (TBA1, TBA2),
dans lequel le modèle, moyennant la modification des valeurs de départ pour les valeurs déterminées des au moins deux caractéristiques physiques du signal électromagnétique (SI2) réceptionné à la deuxième position (P2), est adapté et
dans lequel les valeurs d'humidité (F1 ; F2) de la multiplicité des zones partielles (TBA1, TBA2) de la zone de sol (BA2 ; BR) sont déterminées en se basant sur le modèle adapté.

14. Système selon la revendication 13, dans lequel le dispositif émetteur et/ou le dispositif récepteur (2 ; 3 ; 42) présente/présentent un dispositif de détermination de position et/ou un dispositif de détermination de temps, notamment un récepteur d'un système de satellite de navigation globale (15 ; 25 ; 35).
